# EUROPEAN PATENT SPECIFICATION

(11) **EP 2 396 404 B1**
(45) Date of publication and mention of the grant of the patent: **04.12.2013**
(21) Application number: 10706141.8
(22) Date of filing: 09.02.2010
(51) Int. Cl.: C12N 15/10, C12N 15/63, C12N 15/64, C12N 15/67

(54) **RNAI BASED SELECTION SYSTEM**
AUSWAHLSYSTEM AUF RNAI-BASIS
Système de sélection à base d'ARNi

(30) Priority: 11.02.2009 EP 09001883
(43) Date of publication of application: 21.12.2011
(73) Proprietor: Qiagen GmbH, 40724 Hilden (DE)
(72) Inventor: HAHN, Peter, 51429 Bergisch Gladbach (DE)
(74) Representative: Roth, Carla
(86) International application number: PCT/EP2010/000788
(87) International publication number: WO 2010/091837

(56) References cited:
- WO-A-02/079467
- WO-A-2004/020631
- WO-A-2004/111190
- WO-A-2005/052167
- WO-A-2005/123925
- WO-A-2006/063786
- HON HUIMING ET AL: "bcl-X-L is critical for dendritic cell survival in vivo" JOURNAL OF IMMUNOLOGY, AMERICAN ASSOCIATION OF IMMUNOLOGISTS, US, vol. 173, no. 7, 1 October 2004 (2004-10-01), pages 4425-4432, XP002421386 ISSN: 0022-1767
- HAHN ET AL: "An siRNA-based system for differential regulation of ectopic gene expression constructs" JOURNAL OF BIOTECHNOLOGY, ELSEVIER SCIENCE PUBLISHERS, AMSTERDAM, NL, vol. 128, no. 4, 6 March 2007 (2007-03-06) , pages 762-769, XP005916237 ISSN: 0168-1656
- 'RNA-Interferenz aus Wikipedia, der freien Enzyklopädie', [Online] pages 1 - 10 Retrieved from the Internet: <URL:http://de.wikipedia.org/wiki/RNA-Inter ferenz> [retrieved on 2012-04-02]
- 'RNAi inducing compound', [Online] Retrieved from the Internet: <URL:http://www.google.de> [retrieved on 2012-04-02]
- HORST IBELGAUFTS: "Gentechnologie von A bis Z, Studienausgabe", 1993, VCH, Weinheim, Ney York, Basel, Cambridge ISBN: 3-527-30008-2 * MCS:; page 328 * * Polylinker; page 380 *
- Pühler; Regitz;Schmid: "Lexikon Biochemie und Molekularbiologie", 2000, Georg Thieme Verlag, Stuttgart, New York ISBN: 3-13-116681-9 * Polylinker (MCS); page 487 *

### SEQUENCE LISTING

<110> QIAGEN GmbH Hahn, Peter
<120> RNAi based selection system
<130> 51 337 K
<150> EP 09 001 883.9
   <151> 2009-02-11
<160> 12
<170> PatentIn version 3.3
<210> 1
   <211> 21
   <212> DNA
   <213> Artificial Sequence
<220>
   <223> target sequence for ubiquitin - sequence shown based on the DNA sequence
<400> 1
   taaaggtttc gttgcatggt a 21
<210> 2
   <211> 21
   <212> DNA
   <213> Artificial Sequence
<220>
   <223> target sequence for ubiquitin - sequence shown based on the DNA sequence
<400> 2
   tcagtaatag ctgaacctgt t 21
<210> 3
   <211> 21
   <212> DNA
   <213> Artificial Sequence
<220>
   <223> target sequence for ubiquitin - sequence shown based on the DNA sequence
<400> 3
   gtgcccagtg atggcattac t 21
<210> 4
   <211> 21
   <212> DNA
   <213> Artificial Sequence
<220>
   <223> target sequence for ubiquitin - sequence shown based on the DNA sequence
<400> 4
   aagtttagaa attacaagtt t 21
<210> 5
   <211> 21
   <212> DNA
   <213> Artificial Sequence
<220>
   <223> target sequence for ubiquitin - sequence shown based on the DNA sequence
<400> 5
   ttcagtcatg gcattcgcag t 21
<210> 6
   <211> 21
   <212> DNA
   <213> Artificial sequence
<220>
   <223> target sequence for ubiquitin - sequence shown based on the DNA sequence
<400> 6
   tactctgcac tatagccatt t 21
<210> 7
   <211> 21
   <212> DNA
   <213> Artificial sequence
<220>
   <223> target sequence for ubiquitin - sequence shown based on the DNA sequence
<400> 7
   ttcagtaata gctgaacctg t 21
<210> 8
   <211> 21
   <212> DNA
   <213> Artificial sequence
<220>
   <223> target sequence for ubiquitin - sequence shown based on the DNA sequence
<400> 8
   taatagctga acctgttcaa a 21
<210> 9
   <211> 21
   <212> DNA
   <213> Artificial Sequence
<220>
   <223> target sequence for ubiquitin - sequence shown based on the DNA sequence
<400> 9
   aaatgttaat aaaggtttcg t 21
<210> 10
   <211> 21
   <212> DNA
   <213> Artificial Sequence
<220>
   <223> target sequence for ubiquitin - sequence shown based on the DNA sequence
<400> 10
   gttaataaag gtttcgttgc a 21
<210> 11
   <211> 21
   <212> DNA
   <213> Artificial sequence
<220>
   <223> target sequence for ubiquitin - sequence shown based on the DNA sequence
<400> 11
   ttaataaagg tttcgttgca t 21
<210> 12
   <211> 21
   <212> DNA
   <213> Artificial Sequence
<220>
   <223> target sequence for ubiquitin - sequence shown based on the DNA sequence
<400> 12
   gtaatagctg aacctgttca a 21

## Description

### FIELD OF THE INVENTION

The present invention relates to a novel selection system suitable for selecting eukaryotic host cells comprising an expression vector which comprises an RNAi selectable marker gene. The invention provides suitable expression vectors, eukaryotic host cells and methods for selecting host cells comprising respective expression vectors. Furthermore, the present invention pertains to methods for performing RNAi rescue experiments as well as to methods for producing polypeptides of interest.

### BACKGROUND OF THE INVENTION

RNA interference (RNAi) has become a widely used tool for functional genomic studies in vertebrate and invertebrates. RNAi works by silencing a gene through homologous short interfering dsRNAs (for example siRNAs), which trigger the destruction of the corresponding mRNA by the RNA-induced silencing complex (RISC). The selective and robust effect of RNAi on gene expression makes it a valuable research tool, both in cell culture and in living organisms. Synthetic dsRNA introduced into cells can induce suppression of specific genes of interest. The effect of these genes on the phenotype of the cells can then be analyzed by studying the effect of the gene silencing. RNAi may also be used for large-scale screens that systematically shut down each gene in the cell, which can help identify the components necessary for a particular cellular process or an event such as for example, cell division.

Due to its advantages, in particular siRNA-mediated RNAi has become an indispensable tool in functional genomic research. Chemically synthesized siRNA reagents that target every gene in a human, mouse and rat genome are available for convenient delivery *in vitro.* Data acquired from RNAi experiments are used to support important conclusions about how genes function.

For the above reasons, the RNA interference pathway is often exploited in experimental biology to study the function of genes in cell culture and *in vivo* in model organisms. Double-stranded RNA is synthesized with a sequence complementary to the target sequence of a gene of interest, usually a 18 to 30mer and introduced into the cell or organism, where it is recognized as exogenous genetic material and activates the RNAi pathway. Using this mechanism, researchers can cause a drastic decrease in the expression of the targeted gene. Studying the effects of this decrease can show the physiological role of the respective targeted gene product. Since RNAi may not necessarily totally abolish expression of the gene, this technique is sometimes referred to as a "knockdown" to distinguish it from "knockout" procedures, in which expression of a gene is entirely eliminated, e.g. by introducing a knock-out mutation in the target gene and thus the DNA.

The ease, speed, and cost-effectiveness have made RNAi the method of choice for loss-of-gene function studies. However, even though RNAi is a valuable tool it has produced a new set of problems in determining the specificity of the outlet phenotype. In order to ensure that the conclusion drawn from a RNAi experiment are accurate, it is important to include proper controls in every RNAi experiment. Such controls strengthen the drawn conclusions and ensure that the performed expression modulating experiments result in the expected silencing. Appropriate experimental controls are thus of utmost importance in order to maximize the value of the generated data.

Recent publications reported off-target effects of RNAi inducing compounds that in addition to the targeted genes led to changes in the expression of other genes on both RNA and protein level. Also, several authors reported the induction of genes involved in the IFN response machinery, further challenging the reliability of RNAi in loss of function studies. Due to these problems, control experiments are therefore of utmost importance to confirm the specificity of an RNAi phenotype. A reliable way to be sure of the specificity of the loss of function phenotype is a rescue experiment. A rescue experiment relies on the expression of the target gene in a form refractory to the RNAi mediating compound (for example siRNA). Therefore, the reintroduced rescue gene should be resistant to the RNAi mediating compound. Ideally, this rescue gene should also be expressed within the physiological range. For important model organisms such as yeast, rescue experiments can be easily achieved by using homologous recombination, thereby ensuring physiological expression of the rescue construct. However, rescue experiments in mammalian cells often raise specific problems. To make the rescue gene refractory to the RNAi mediating compound, one or more silent third-codon point mutations can be introduced within the targeted region, although controls for fortuitous mRNA are desirable. Translational effects can be avoided by utilizing siRNAs targeted against the 3' untranslated regions (UTRs) which are non-essential for the rescue expression from a plasmid/vector.

Therefore, RNAi rescue experiments are used as inevitable controls in RNAi experiments. Thereby, it can be analysed whether the wildtype phenotype is restored and accordingly, whether the RNAi mediating compound is specific for the target gene and accordingly the observed RNAi phenotype is attributable to the silencing of the corresponding target gene. However, when doing respective rescue-experiments to verify the specificity of siRNAs, co-transfection of the siRNA and the corresponding recombinantly expressed plasmid-derived protein is necessary. The low plasmid-transfection rate is complicating the analysis of the rescue phenotype, because the population of cells transfected with siRNA only is often higher than the one transfected with both plasmid and siRNA. Due to this fact, measuring the reverted phenotype is more or less impossible without using highly sophisticated cell based assays or using technologies to enrich the plasmid-transfected cells.

Therefore, it is one object of the present invention to improve the existing RNAi rescue experiments.

Furthermore, the selection of host cells, which have incorporated an expression vector comprising a polynucleotide encoding a polypeptide of interest is of importance for several applications, in particular the expression of products of interest e.g. on an experimental or industrial scale. The ability to clone and express products of interest such as recombinant peptides and proteins has become increasingly important. The ability to purify high levels of proteins is important in the human pharmaceutical and biotechnological field, for example for producing protein pharmaceuticals as well as in the basic research setting, for example for crystallizing proteins to allow the determination of their three dimensional structure. Proteins that are otherwise difficult to obtain in quantity can be over-expressed in a host cell and subsequently isolated and purified.

Hon et al (bcl-xL is critical for dendritic cell survival in vivo; the Journal of Immunology, page 4425-4432; 2004) analyses the role of bcl-x_{L} for dendritic cell survival in vivo. In the course of the performed analysis, cells are co-transfected with a plasmid expressing a bcl-x_{L} -siRNA and plasmids expressing either bcl-x_{L} or bcl-x_{S}. However, no eukaryotic selection system is disclosed therein that is based on the use of bcl-x_{L} and/or siRNA compounds.

WO 02/079467 discloses an antibiotic-free bacterial strain selection system that uses antisense molecules for selection. In said method which is suitable for selecting genetically modified cells, a vector containing a gene Y is introduced into cells which contain a growth essential gene X. The respectively modified cells are treated with an antisense or antigene construct directed against the essential gene X. Thereby, modified cells can be obtained which show preferential growth over other, non-modified cells. As examples for respective antisense or antigene constructs, said document teaches the use of oligonucleotides, DNA minor groove binding polyamides, PNAs, LNAs, morpholinos and the like. The preferred antisense compound used is a PNA.

WO 2005/123925 discloses an RNAi-based method for selecting transfected eukaryotic cells. In said method, a vector is introduced into the cells, wherein a siRNA is expressed from the respective vector which targets an endogenous, selectable gene and inactivates said gene. Preferably, the endogenous selectable gene is selected from the group consisting of HPRT, APRT, DHFR and TK.

WO 2006/063786 discloses a method for inactivation of expression of a gene in a eukaryotic cell comprising transforming a eukaryotic cell with a DNA comprising an expression cassette for expression of a cell surface protein and an expression cassette for expression of an RNAi compound wherein said compound is being capable of inactivating the expression of said gene. The cells which express said cell surface protein are enriched/selected.

The choice of an expression system for the production of recombinant proteins depends on many factors, including cell growth characteristics, expression levels, intracellular and extracellular expression, post-translational modifications and biological activity of the protein of interest, as well as regulatory issues and economic considerations in the production of therapeutic proteins. Key advantages of mammalian cells over other expression systems such as bacteria or yeast are the ability to carry out proper protein folding, complex N-linked glycosylation and authentic O-linked glycosylation, as well as a broad spectrum of other post-translational modifications. Due to the described advantages, eukaryotic and in particular mammalian cells are currently the expression system of choice for producing complex therapeutic proteins such as monoclonal antibodies.

The most common approach to obtain expressing host cells generates an appropriate expression vector for expressing the product of interest as a first step. The expression vector drives the expression of the polynucleotide encoding the product of interest in the host cell and provides at least one selectable marker for generating the recombinant cell line. Key elements of mammalian expression vectors usually include a constitutive or inducible promoter capable of robust transcriptional activity; optimized mRNA processing and translational signals that usually include a Kozak sequence, a translation termination codon, mRNA cleavage and polyadenylation signals, a transcription terminator and selectable markers for the preparation of stable cell lines and if desired for gene amplification; furthermore a prokaryotic origin of replication and selectable markers for vector propagation in bacteria can be provided by the expression vector.

In recent years the focus of development was concentrating on the design of improved vectors for gene expression in host and in particular in mammalian cells. Despite of the plethora of available vectors, however, robust polypeptide/protein production with a high yield in mammalian cells is still challenging.

Selectable markers and selection systems are widely used in genetic engineering, recombinant DNA technology and the production of recombinant products in order to obtain eukaryotic host cells expressing the product of interest with high yield. Respective systems are also useful to generate and identify stably or transiently transfected clones. The primary goal of using respective selectable markers and selection systems is to introduce a selectable gene which upon exposure to selective growth conditions allows the identification of cells which have incorporated the expression vector and which are accordingly capable of producing the recombinant products of interest. Several selection systems/selectable markers are known and in use for eukaryotes, thereunder dihydrofolate reductase (DHFR) or glutamine synthetase (GS), neomycin, hygromycin and the like. Some of these markers such as DHFR also allow the amplification of the genes when exposed to the selective culturing conditions. The aim of providing such a selection pressure is to isolate cells that express the selectable markers and accordingly, the product of interest with a high yield.

However, the known selection systems also have drawbacks. The selection conditions (e.g. when using antifolates such as MTX in case of DHFR) are to a certain extent toxic for the host cell and may e.g. alter the genome of the host cell. Also antibiotic based expression systems have drawbacks.

Therefore, it is also the object of the present invention to provide an alternative selection system for selecting eukaryotic host cells that have incorporated an expression vector, as well as to provide suitable expression vectors and eukaryotic host cells.

### SUMMARY OF THE INVENTION

The present invention pertains to a novel selection system suitable for selecting eukaryotic host cells that were successfully transfected with an expression vector.

According to one embodiment, a method for selecting a eukaryotic host cell comprising an introduced expression vector is provided, said method comprising the following steps:
a) Providing a population of eukaryotic host cells, wherein said host cells endogenously express at least one gene essential for cell survival;
b) Introducing an expression vector into said eukaryotic host cells, wherein said expression vector comprises at least one recombinant polynucleotide encoding a product which corresponds to and/or is a functional variant or functional equivalent of the product encoded by said gene essential for cell survival that is endogenously expressed by said eukaryotic host cells;
c) Culturing the eukaryotic host cells under selective conditions, wherein the selective conditions comprise at least the introduction of at least one RNAi inducing compound into said eukaryotic host cells, wherein said RNAi inducing compound targets at least said gene essential for cell survival that is endogenously expressed by said eukaryotic host cells and wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

To allow selection, the expression vector comprises a recombinant polynucleotide encoding a product which corresponds to and/or is a functional variant or functional equivalent of the product of a gene essential for cell survival that is endogenously expressed by said eukaryotic host cells. Eukaryotic host cells that have incorporated said expression vector are resistant to the applied selective culture conditions, wherein at least one RNAi inducing compound (as defined) targeting at least said gene essential for cell survival that is endogenously expressed by said host cells is introduced into the host cells. Eukaryotic host cells that were successfully transfected comprise said expression vector and express said recombinant polynucleotide. Therefore, they can survive the selective culture conditions as said recombinant polynucleotide encoding a product which corresponds to and/or is a functional variant or functional equivalent of the product of said endogenously expressed gene essential for cell survival takes over the function of said endogenously expressed gene essential for cell survival that is knocked-down by the RNAi inducing compound used for selection. The expression of said recombinant polynucleotide basically restores the function of the endogenously expressed essential gene that is knocked down by the selective RNA inducing compound. Host cells that were not successfully transfected with the expression vector are not able to survive/proliferate under the selective culture conditions as the endogenously expressed gene essential for cell survival is knocked down by the selective RNAi inducing compound, thereby preferably inducing apoptosis in untransfected eukaryotic host cells which are thus eliminated from the culture. Thus, a novel RNAi based selection system for identifying eukaryotic host cells that have incorporated an expression vector is provided by the teachings of the present invention.

According to a further embodiment, a method for producing a product of interest, in particular a polypeptide is provided, comprising culturing a eukaryotic host cell selected according to the teachings of the present invention under conditions that allow for the expression of the product of interest.

According to a further embodiment, a method for performing an RNAi experiment is provided, said method comprising the following steps:
a) introducing an expression vector into a eukaryotic host cell which endogenously expresses a gene essential for cell survival, wherein said expression vector comprises at least
   i) a recombinant polynucleotide encoding a product which corresponds to and/or is a functional variant or functional equivalent of the product of a gene essential for cell survival that is endogenously expressed by said eukaryotic host cell;
   ii) a recombinant polynucleotide encoding a product which corresponds to and/or is a variant or equivalent of the product of a target gene of interest expressed by said eukaryotic host cell;
b) introducing into said eukaryotic host cell a RNAi inducing compound targeting the expression of said target gene of interest expressed by said eukaryotic host cell, wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound;
c) culturing the eukaryotic host cells under selective conditions, wherein the selective conditions comprise at least the introduction of at least one RNAi inducing compound, wherein said RNAi inducing compound targets at least the gene essential for cell survival that is endogenously expressed by said eukaryotic host cell and wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

Also provided by the teachings of the present invention is a kit, wherein said kit comprises at least:
a) an expression vector to be introduced into a eukaryotic host cell which comprises
   (i) an expression cassette comprising a recombinant polynucleotide encoding a product which corresponds to and/or is a functional variant or functional equivalent of a gene essential for cell survival that is endogenously expressed by said eukaryotic host cell; and
   (ii) a further expression cassette comprising a multiple cloning site for inserting a recombinant polynucleotide of interest;
b) an RNAi inducing compound targeting said gene essential for cell survival that is endogenously expressed by said eukaryotic host cell, wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

### DETAILED DESCRIPTION OF THE INVENTION

According to one aspect of the present invention, a method for selecting a eukaryotic host cell comprising an introduced expression vector is provided, said method comprising at least the following steps:
a) Providing a population of eukaryotic host cells, wherein said eukaryotic host cells endogenously express at least one gene essential for cell survival;
b) Introducing an expression vector into said eukaryotic host cells, wherein said expression vector comprises at least one recombinant polynucleotide encoding a product which corresponds to and/or is a functional variant or functional equivalent of the product encoded by said gene essential for cell survival that is endogenously expressed by said eukaryotic host cells;
c) Culturing the eukaryotic host cells under selective conditions, wherein the selective conditions comprise at least the introduction of at least one RNAi inducing compound into said host cells, wherein said RNAi inducing compound targets at least said gene essential for cell survival that is endogenously expressed by said eukaryotic host cells and wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

An "expression vector" according to the present invention in particular refers to a polynucleotide capable of carrying at least one recombinant polynucleotide. A vector functions like a molecular carrier, delivering polynucleotides or fragments thereof into a host cell. It may comprise at least one expression cassette comprising regulatory sequences for properly expressing a polynucleotide incorporated therein. Polynucleotides (e.g. encoding a product of interest or a selectable marker) to be introduced into the cell may be inserted into the expression cassette(s) of the vector in order to be expressed therefrom. The vector according to the present invention may be present in circular or linear(ized) form and the term "vector" also encompasses vector fragments as well as artificial chromosomes or similar respective polynucleotides allowing the transfer of foreign nucleic acid fragments.

A "polynucleotide" in particular refers to a polymer of nucleotides which are usually linked from one deoxyribose or ribose to another and refers to DNA as well as RNA, depending on the context. The term "polynucleotide" does not comprise any size restrictions and also encompasses polynucleotides comprising modifications, in particular modified nucleotides.

A "recombinant polynucleotide" in particular refers to a polynucleotide that has been or is intended to be introduced into a eukaryotic host cell e.g. by the use of recombinant techniques such as transfection and/or transformation. The terms "transfection" and "transformation" are herein used as synonyms. Depending on the embodiment, the eukaryotic host cell may or may not comprise an endogenous polynucleotide corresponding to, respectively being identical to the recombinant polynucleotide. Introduction may be achieved e.g. by transfecting a suitable vector that may integrate into the genome of the eukaryotic host cell (stable transfection). Suitable vectors allowing the introduction of polynucleotides into the eukaryotic host cell are described in detail below and are also known in the prior art. In case the introduced polynucleotide is not inserted into the genome it is also referred to as a transient transfection. When a transient transfection is performed, the introduced polynucleotide can be lost at the later stage e.g. when the cells undergo mitosis. However, suitable vectors might also be maintained in the eukaryotic host cell without integrating into the genome, e.g. by episomal replication. Also other techniques are known in the prior art for introducing a polynucleotide into a eukaryotic host cell and some of them are also described in further detail below.

A "gene essential for cell survival" in particular refers to a gene that is involved in essential processes of the cell and in particular is involved in the cell metabolism. A gene essential for cell survival is in particular a gene that is relevant respectively essential for the proper maintenance of the cell viability at least under certain culture/growth conditions. Said term in particular includes but is not limited to a gene involved in the cell cycle, apoptosis, cell division, DNA transcription, replication and repair or cell differentiation and development. The down-regulation, respectively silencing of a respective gene essential for cell survival by RNAi induces, results in or promotes cell death, in particular apoptosis and/or inhibits cell growth/cell proliferation. A respective gene is endogenously expressed by said eukaryotic host cell. The term "gene" also includes functional variants or fragments of a gene. Suitable examples of a gene essential for cell survival are described in detail below.

The term "endogenously expressed" is in particular used in order to differentiate genes, respectively polynucleotides that are expressed from the expression vector according to the present invention from genes that are expressed from the eukaryotic host cells. Respective endogenously expressed genes are preferably naturally expressed by said eukaryotic host cells but many also have been introduced into said eukaryotic host cells by recombinant techniques, e. g. by using a vector. The decisive characteristic of an endogenously expressed gene is that it is not expressed from the expression vector according to the present invention.

The term a "product which corresponds to and/or is a functional variant or functional equivalent of the product of said gene essential for cell survival that is endogenously expressed by said host cell" in particular refers to a product capable of restoring the function of the product of said gene essential for cell survival that is endogenously expressed by said eukaryotic host cell when said gene is knocked-down according to the teachings of the present invention. Hereinafter, we also refer to said product as the "restoration product". It is encoded by the recombinant polynucleotide comprised in the expression vector. Hereinafter, we also refer to said recombinant polynucleotide as the "restoration polynucleotide". The expression of said product which corresponds to and/or is a functional variant or functional equivalent of the product of said gene essential for cell survival that is endogenously expressed by said host cell (the restoration product) is capable of restoring and/or maintaining the cell viability of the host cell in case said gene essential for cell survival endogenously expressed by said host cell is targeted and thus down-regulated by the RNAi inducing compound used for selection. Therefore, the recombinant polynucleotide encoding a product which corresponds to and/or is a functional variant or functional equivalent of the product of said gene essential for cell survival that is endogenously expressed by said host cell (the restoration polynucleotide) protects the eukaryotic host cell against the selection with a RNAi inducing compound targeting said gene essential for cell survival that is endogenously expressed by said eukaryotic host cell. Accordingly, the restoration polynucleotide basically functions like a RNAi selectable marker gene protecting those cells against the selection conditions that have successfully incorporated the expression vector and accordingly enables the selection of those eukaryotic host cells which comprise the expression vector. Examples of restoration polynucleotides include but are not limited to polynucleotides comprising the identical coding sequence as the endogenously expressed gene essential for cell survival, polynucleotides encoding the same or a homologous product that are derived from a different species as well as functional variants and/or equivalents of the foregoing which include but are not limited to polynucleotides encoding products comprising one or more amino acid mutation(s), substitutions, deletions and/or additions as well as functional fusion products. The decisive characteristic is that said restoration polynucleotide is capable of restoring the function of the endogenously expressed gene essential for cell survival in case said gene is knocked down during selection.

The term "selective conditions" or "selective culture conditions" interchangeably in particular refer to culture conditions, wherein a selection pressure is exerted onto the host cells. The selective culture conditions benefit the survival of eukaryotic host cells that have incorporated the expression vector according to the present invention as eukaryotic host cells comprising said vector have a selective advantage due to the expression of the restoration polynucleotide. According to the teachings of the present invention, the selective conditions comprise at least the introduction of at least one RNAi inducing compound into the eukaryotic host cells, wherein said RNAi inducing compound targets at least said gene essential for cell survival that is endogenously expressed by said eukaryotic host cell thereby exerting a selection pressure onto the eukaryotic host cell.

The present invention pertains to a novel RNAi based selection system suitable for selecting eukaryotic host cells that were successfully transfected with an expression vector. Said eukaryotic host cells endogenously express a gene essential for cell survival. To allow selection, the expression vector comprises a recombinant polynucleotide encoding a product which corresponds to and/or is a functional variant or functional equivalent of the product of said gene essential for cell survival that is endogenously expressed by said eukaryotic host cells. Host cells that have incorporated said expression vector are due to the comprised restoration polynucleotide resistant to the applied selective culture conditions. For selection, at least one RNAi inducing compound targeting at least said gene essential for cell survival that is endogenously expressed by said eukaryotic host cells is introduced into the eukaryotic host cells. Thus, the endogenously expressed gene essential for cell survival is knocked-down by the introduced RNAi inducing compound. Host cells that were successfully transfected comprise said expression vector and which accordingly express the restoration polynucleotide can survive the selective culture conditions as the encoded restoration product takes over the function of said endogenously expressed gene essential for cell survival that is knocked-down by the RNAi inducing compound.

Therefore, the RNAi induced knock-down of the endogenously expressed gene essential for cell survival is compensated by the restoration polynucleotide comprised in the expression vector thereby allowing the eukaryotic host cells to survive and preferably to proliferate under the selective culture conditions. However, eukaryotic host cells that were not successfully transfected with the expression vector are not able to survive/proliferate under the selective culture conditions as the endogenously expressed gene essential for cell survival is knocked down by the RNAi inducing compound, thereby preferably eliminating untransfected host cells. Thus, the recombinant polynucleotide encoding a product which corresponds to and/or is a functional variant or functional equivalent of the product of said endogenously expressed gene essential for cell survival basically functions as an RNAi selectable marker gene allowing the selection of host cells that have incorporated the expression vector under selective culture conditions. Thus, a novel RNAi based selection system for identifying and selecting eukaryotic host cells that have incorporated an expression vector is provided by the teachings of the present invention.

The selection system according to the present invention has several advantages. E.g. no toxic substances such as antifolates or antibiotics are used for selection, which have a certain risk to alter the eukaryotic host cells. The selective pressure is created by the use of an RNAi inducing compound which targets and accordingly knocks down an endogenously expressed gene essential for cell survival. Said knock-down is reversible as soon as the RNAi inducing compound is removed/no longer introduced into the eukaryotic host cell. Furthermore, the selection system according to the present invention allows the use of wildtype eukaryotic host cells if desired.

According to one embodiment, at least one eukaryotic host cell is selected that survives the selective growth conditions. It is also possible to select a population of eukaryotic host cell surviving the selective growth conditions for further use. Further uses include e.g. for example the analysis of the phenotype e.g. in a RNAi rescue experiment and/or the use of the respectively selected eukaryotic host cell for producing a product of interest. It is also within the scope of the present invention, to perform several rounds of selection.

A "product of interest" in particular refers to a product to be expressed from said expression vector. The product of interest may be e.g. a polypeptide or a polynucleotide, such as e.g. a RNA. Preferably, the product of interest is a polypeptide. Further examples are described below.

Preferably, the eukaryotic host cells that did not incorporate efficiently the expression vector do not survive the selective culture conditions and e.g. undergo apoptosis. Depending on the transfection method/system, for example, a successful transient or stable transfection at least needs to ensure that the recombinant polynucleotide encoding a product which corresponds to and/or is a functional variant or functional equivalent of the product encoded by said gene essential for cell survival that is endogenously expressed by said eukaryotic host cells is expressed with sufficient yield by the eukaryotic host cells in order to survive the selective growth conditions. Host cells that do not meet these criteria preferably die under selective growth conditions. This embodiment has the advantage that only those host cells grow and are therefore present in the culture, which have successfully incorporated the expression vector. Therefore, when performing, for example, an RNAi rescue experiment (see above and below), and/or when performing a selection procedure to select a eukaryotic host cell expressing and thus producing a product of interest, it can easily be determined by using the RNAi based selection system according to the present invention whether the expression vector has been introduced successfully into the eukaryotic host cell.

The induction of cell death is efficient in eliminating eukaryotic host cells that have not incorporated the expression vector. In this respect, the selection system according to the present invention is similar to conventional selection systems which are based on the use of toxic substances such as antifolates or antibiotics. However, as a RNAi inducing compound is used in order to exert the selection pressure the risk of permanent, respectively irreversible damages of the successfully transfected eukaryotic host cells is considerably reduced as the normal cell function is usually restored as soon as the RNAi inducing compound that down-regulates the endogenously expressed gene essential for cell survival is eliminated from the culture medium (see also above). Furthermore, apoptosis/cell death is a phenotype which is rather easy to determine e.g. via microscopic techniques. In particular in the case of adherent cells, dying (apoptotic or necrotic) cells change their morphology in a very characteristic way, as they round up and detach from the surface they adhered to. Therefore, if desired, it can also be easily visually determined whether the eukaryotic host cells have incorporated the expression vector.

In order to further increase the selection pressure, the selective conditions may comprise the introduction of at least one further selective RNAi inducing compound which targets a second gene essential for cell survival that is also endogenously expressed by said eukaryotic host cell. In this embodiment, the expression vector comprises a further recombinant polynucleotide encoding a product which corresponds to and/or is a functional variant or functional equivalent of the product of said second gene essential for cell survival that is endogenously expressed by said eukaryotic host cell. This embodiment has the advantage that due to the targeting of at least two genes essential for cell survival the selection pressure is increased, thereby providing very stringent selection conditions. Thus, untransfected cells are more quickly killed, thereby reducing the time necessary for selection.

According to one embodiment, the recombinant polynucleotide encoding a product which corresponds to and/or is a functional variant or functional equivalent of said gene essential for cell survival that is endogenously expressed by said host cell (the restoration polynucleotide) is an RNAi selectable marker gene. An RNAi selectable marker gene in particular refers to a gene which provides resistance to an RNAi inducing compound introduced into the host cell and which allows the selection of eukaryotic host cells that have incorporated an expression vector under the selection conditions. Therefore, under selective culture conditions said RNAi selectable marker gene promotes/benefits cell survival of the eukaryotic host cells that have incorporated the expression vector according to the present invention.

According to a further embodiment, the expression vector comprises a polynucleotide encoding a product of interest. This embodiment is advantageous for several applications/uses of the RNAi based selection system according to the present invention. For example, when performing RNAi rescue experiments, the polynucleotide encoding the product of interest may be the rescue gene that may restore the function of the endogenously expressed target gene that is down regulated in the RNAi expression experiment. In a RNAi rescue experiment, the expression of the product of interest from the expression vector according to the present invention aims at the restoration of the original/wildtype phenotype in case the RNAi inducing compound that was used for performing the RNAi experiment was sufficiently specific for the respective target gene. Thereby, a very valuable control for performing RNAi experiments is provided and due to the RNAi based selection system which targets a cell essential for cell survival it is also ensured that the host cell that is analysed for the rescued/restored phenotype has incorporated the expression vector that is essential for performing the respective control. Therefore, the selection method according to the present invention is an improved control for a RNAi rescue experiments.

According to a further embodiment, the expression vector comprises a recombinant polynucleotide encoding a product of interest that is supposed to be expressed from the eukaryotic host cells, e.g. for the experimental or industrial production of said product of interest. As is outlined in the introduction, the expression and, in particular, the over-expression of products of interest, in particular polypeptides, is of particular interest for several applications, for example in order to produce polypeptides that can be used/analysed in science and/or industry, for example as therapeutic polypeptides. An expression vector comprising a recombinant polynucleotide encoding a product which corresponds to and/or is a functional variant or functional equivalent of said product encoded by the endogenously expressed gene essential for cell survival as an RNAi selectable marker gene and comprising a recombinant polynucleotide encoding a product of interest, for example a polypeptide, provides a valuable and novel expression vector that can be selected based on the RNAi mechanism.

Expression vectors used for expressing an incorporated polynucleotide usually contain transcriptional control elements suitable to drive the transcription of said polynucleotide. Respective transcriptional control elements include but are not limited to e.g. promoters, enhancers, polyadenylation signals, transcription pausing or termination signals. The respective elements often form part of an expression cassette. If the desired product is a polypeptide, suitable translational control elements are preferably included, such as e.g. 5' untranslated regions leading to 5' cap structures suitable for recruiting ribosomes and stop codons to terminate the translation process. A functional expression unit, capable of properly driving the expression of an incorporated polynucleotide is also referred to as an "expression cassette" herein. The recombinant polynucleotide(s) of the expression vector are preferably comprised in an expression cassette. Several embodiments are suitable, for example each polynucleotide can be comprised in a different expression cassette. However, it is also within the scope of the present invention that at least two of the respective polynucleotides are comprised in one expression cassette. Therefore, the present invention encompasses mono-as well as bicistronic embodiments.

The expression vector according to the present invention may comprise at least one promoter and/or enhancer element as element of an expression cassette. Although the physical boundaries between these two control elements are not always clear, the term "promoter" usually refers to a site on the nucleic acid molecule to which an RNA polymerase and/or any associated factors binds and at which transcription is initiated. Enhancers potentiate promoter activity, temporally as well as spatially. Many promoters are transcriptionally active in a wide range of cell types. Promoters can be divided in two classes, those that function constitutively and those that are regulated by induction or derepression. Promoters used for the production of polypeptides in mammalian cells should be strong and preferably active in a wide range of cell types. In case the restoration polynucleotide is only to be expressed during selection, it is preferred to use an inducible promoter that is induced and accordingly active under the selective growth conditions.

Strong constitutive promoters which drive the expression in many cell types include but are not limited to the adenovirus major late promoter, the human cytomegalovirus immediate early promoter, the β-actin promoter, the SV40 and Rous Sarcoma virus promoter, and the murine 3-phosphoglycerate kinase promoter, EF1a. Good results are achieved with the expression vector of the present invention when the promoter and/or enhancer are either obtained from CMV, β-actin and/or SV40. The transcription promoters can be selected from the group consisting of an SV40 promoter, a CMV promoter, an EF1 alpha promoter, a RSV promoter, a BROAD3 promoter, a murine rosa 26 promoter, a pCEFL promoter and a β-actin promoter.

According to one embodiment, the recombinant polynucleotide encoding a product which corresponds to and/or is a functional variant or functional equivalent of the product encoded by said gene essential for cell survival that is endogenously expressed by said eukaryotic host cells, the polynucleotide encoding the product of interest and/or the recombinant polynucleotide encoding a product which corresponds to and/or is a functional variant or functional equivalent of the product encoded by the second gene essential for cell survival that is endogenously expressed by said eukaryotic host cells are under the control of the same transcription promoter. Suitable promoters are described above. In this embodiment, one long transcript is obtained from the respective expression cassette that is under the control of said transcription promoter. According to one embodiment, at least one IRES element is functionally located between the recombinant polynucleotides. Thereby, it is ensured that separate translation products are obtained from said transcript. However, preferably the individual polynucleotides are expressed from separate expression cassettes.

Furthermore, the expression vector may comprise an appropriate transcription termination site as element of an expression cassette. This, as continued transcription from an upstream promoter through a second transcription unit may inhibit the function of the downstream promoter, a phenomenon known as promoter occlusion or transcriptional interference. This event has been described in both prokaryotes and eukaryotes. The proper placement of transcriptional termination signals between two transcription units/expression cassettes can prevent promoter occlusion. Transcription termination sites are well characterized and their incorporation in expression vectors has been shown to have multiple beneficial effects on gene expression.

Most eukaryotic nascent mRNAs possess a poly A tail at their 3' end which is added during a complex process that involves cleavage of the primary transcript and a coupled polyadenylation reaction. The polyA tail is advantageous for mRNA stability and transferability. Hence, the expression cassettes of the vector according to the present invention usually comprise a polyadenylation site. There are several efficient polyA signals that can be used in mammalian expression vectors, including those derived from bovine growth hormone (bgh), mouse beta-globin, the SV40 early transcription unit and the Herpes simplex virus thymidine kinase gene. However, also synthetic polyadenylation sites are known. The polyadenylation site can be selected from the group consisting of SV40polyA site, such as the SV40 late and early poly-A site (see e.g. plasmid pSV2-DHFR as described in Subramani et al, 1981, Mol.Cell. Biol. 854-864), a synthetic polyA site and a bgh polyA site (bovine growth hormone).

Furthermore, the expression cassette comprising a recombinant polynucleotide (e.g. encoding a product of interest or a restoration polynucleotide) may comprise at least one intron. This embodiment is particularly suitable when a mammalian host cell is used for expressing a product of interest. Most genes from higher eukaryotes contain introns which are removed during RNA processing. Respective constructs are expressed more efficiently in transgenic systems than identical constructs lacking introns. Usually, introns are placed at the 5' end of the open reading frame but may also be placed at the 3' end. Accordingly, an intron may be comprised in the expression cassette(s) to increase the expression rate. Said intron may be located between the promoter and or promoter/enhancer element(s) and the 5' end of the open reading frame of the polynucleotide to be expressed. Several suitable introns are known in the state of the art that can be used in conjunction with the present invention.

In case the expression vector is used for expressing a product of interest that is supposed to be harvested from the culture media, the expression cassette may comprise a secretory leader sequence which directs the expressed product of interest into the culture media. Several suitable leader sequences are known in the prior art.

In order to allow the replication of the expression vector in prokaryotes and/or eukaryotes, the expression vector may comprise appropriate origin(s) of replication.

Furthermore, the expression vector according to the present invention may additionally comprise one or more further polynucleotide(s) encoding one or more additional selectable marker(s). In one embodiment of the present invention co-selection utilizing the system of the present invention together with one or more different selection system(s) (e.g. neo/G418 or DHFR/antifolate or the glutamine synthetase system) can be applied to further improve the performance. The additional selectable marker can be a "eukaryotic selectable marker" and thus a selectable marker that *inter alia* allows the selection of eukaryotic host cells under appropriate selection conditions; said marker may be selectable by antibiotics. Said eukaryotic selectable marker may also be an amplifiable marker. Suitable systems are known in the prior art. Besides further eukaryotic selectable markers, also prokaryotic selectable markers can be used. A "prokaryotic selectable marker" is a selectable marker allowing the selection of prokaryotic host cells under appropriate selection conditions. Examples of respective prokaryotic selectable markers are markers which provide a resistance to antibiotics such as e.g. ampicillin, kanamycin, tetracycline and/or chloramphenicol and are also known in the prior art.

The expression vector according to the present invention can be transfected into the eukaryotic host cell in its circular or linear(ized) form.

As is outlined above, an RNAi inducing compound (as defined) is used in order to exert a selective pressure onto the transfected host cell (herein also referred to as "selective RNAi inducing compound"). The RNAi inducing compound that is used for the selective culture conditions targets a gene essential for cell survival that is endogenously expressed by said eukaryotic host cells. As is outlined above, it is also possible to use at least two or more RNAi inducing compounds as defined which preferably target different genes essential for cell survival that are endogenously expressed by the eukaryotic host cells. By targeting different essential genes, the selection pressure is considerably increased. As is outlined above, the expression vector then comprises two corresponding restoration polynucleotides. Furthermore, it is also possible that at least two different RNAi inducing compounds are used which target the same essential gene but e.g. use different target sites in order to efficiently induce the knock-down of said gene.

Preferably, the RNAi inducing compound used for selection does not target the transcript of the corresponding restoration polynucleotide comprised in the expression vector. According to this preferred embodiment, the respective RNAi inducing compound used for exerting the selection pressure only targets the gene essential for cell survival that is endogenously expressed by the eukaryotic host cell but not the corresponding restoration gene comprised in the expression vector. Hence, according to a preferred embodiment, the restoration polynucleotide is resistant to and accordingly is not targeted by the RNAi inducing compound that is used for selection. There are several ways to obtain such a selective targeting profile. According to one embodiment, mismatches are provided in the corresponding target sequence of the restoration polynucleotide, making the restoration polynucleotide refractory to the selective RNAi inducing compound. Suitable methods are known in the prior art (please also refer to the introduction).

Furthermore, functional equivalents of said gene essential for cell survival e.g. from a different species can be used as restoration polynucleotide which do not show a sufficient homology in the target sequence of the endogenously expressed gene to allow a knock-down of the expression of the restoration polynucleotide by the selective RNAi inducing compound.

According to a further embodiment said RNAi inducing compound used for selection targets an untranslated region (UTR) of the expression product of said gene essential for cell survival that is endogenously expressed by said eukaryotic host cells, preferably the 3' UTR of the transcript. In order to allow the selective targeting of the endogenously expressed gene, said RNAi inducing compound may target an untranslated region of said gene essential for cell survival that is endogenously expressed by said eukaryotic host cells. Preferably, said untranslated region is not present in the corresponding restoration polynucleotide and thus the recombinant polynucleotide encoding a product which corresponds to and/or is a functional variant or functional equivalent of said gene essential for cell survival that is endogenously expressed by said eukaryotic host cell. Thereby, the restoration polynucleotide becomes refractory and accordingly resistant to the selection conditions. A respective setting can be easily achieved by appropriately designing the expression vector.

The RNAi inducing compounds that is used according to the teachings of the present invention is selected from short interfering nucleic acids (siNA), short interfering RNA (siRNA), microRNA (miRNA) and short hairpin RNAs (shRNA) as well as precursors thereof which are processed in the cell to the actual RNAi inducing compound. Preferably, said compound is a siRNA. As siRNA, said compound is a double-stranded molecule preferably having 3' overhangs on each strand. Said siRNA compound may comprise desoxy- as well as ribonucleotides and furthermore, modified nucleotides. Several embodiments and variations of siRNA compounds are known in the prior art and can be used in conjunction with the present invention. The length of said siRNA is usually between 18 and 35 nt, preferably between 19 and 27 nt. The 3' overhangs on each end if present are preferably 2 nts long, but blunt ended molecules may also be used. In order to efficiently induce silencing, the siRNA used as RNAi inducing compound is substantially complementary to a portion of the target gene transcript for inhibiting the expression of said target transcript by RNA interference. Suitable siRNAs targeting the chosen/identified target sequences of the target genes on the RNA level can be identified by using proper computational methods, applying certain design-algorithms. Several methods are known and can be used in conjunction with the present invention in order to provide suitable siRNAs.

In order to obtain a siRNA of the above structure against the target transcript, the double-stranded molecule can be transfected directly into the cell. Alternatively, this structure may result by processing by dicer, an enzyme that converts either long dsRNAs or small hairpin RNAs (shRNAs) into siRNAs (see above). These precursors or the final siRNA molecules can be produced exogenously (artificially) and can then be introduced into the cells to be analyzed by various transfection methods, to analyze the specific knockdown of the target genes involved in apoptosis.

According to a further embodiment, the RNAi inducing compound is expressed by a vector. This embodiment is advantageous, as e.g. transfection of an exogenous siRNA can be sometimes problematic, as the gene knockdown effect is only transient, particularly in rapidly dividing cells. One way of overcoming this challenge is to modify the RNAi inducing compound in such a way as to allow it to be expressed by an appropriate vector, for example a plasmid. For siRNA, this is done by the introduction of a loop between the two strands, thus producing a single transcript, which can be then processed into a functional siRNA in the cell. Such transcription cassettes typically use an RNA polymerase 3 promoter (for example U6 or H1) which usually direct the transcription of small nuclear RNAs (shRNAs) (U6 is involved in gene's placing; H1 is the RNA subcomponent of human RNAse p). It is assumed that the resulting shRNA transcript from the vector is then processed by dicer, thereby producing the double-stranded siRNA molecules, preferably having the characteristic 3' overhangs.

The host cell is a cell which is susceptible to RNA interference, and accordingly is a eukaryotic host cell. Said eukaryotic cell is, preferably, selected from the group consisting of a mammalian cell, an insect cell, a plant cell and a fungi cell. Preferably, the eukaryotic host cell is an insect or a mammalian cell. A mammalian cell is preferably selected from the group consisting of a rodent cell, a primate cell such as a human cell or a monkey cell. Particularly preferred is a rodent cell, which preferably is selected from the group consisting of a CHO cell, a BHK cell, a NS0 cell, a mouse 3T3 fibroblast cell, and a SP2/0 cell. Also preferred is a human cell, which, preferably, is selected from the group consisting of a HEK293 cell, a MCF-7 cell, a PerC6 cell, and a HeLa cell. Further preferred is a monkey cell, which, preferably, is selected from the group consisting of a COS-1, a COS-7 cell and a Vero cell.

There are several appropriate methods known in the prior art for introducing polynucleotides and accordingly an expression vector into a eukaryotic host cell. Respective methods include but are not limited to calcium phosphate transfection, electroporation, lipofection, the use of transfection reagents such as cationic lipids, biolistic- and polymer-mediated transfers. In case of a stable transfection, besides traditional random integration based methods also recombination mediated approaches can be used to transfer the expression vector into the host cell genome. Such recombination methods may include use of site specific recombinases like Cre, Flp or ΦC31 (see e.g. Oumard et al, Cytotechnology (2006) 50: 93 - 108) which can mediate directed insertion of transgenes. Alternatively, the mechanism of homologous recombination might be used to insert said polynucleotides (reviewed in Sorrell et al, Biotechnology Advances 23 (2005) 431 - 469). Recombination based gene insertion allows to minimize the number of elements to be included in the heterologous nucleic acid that is transferred/introduced to the eukaryotic host cell. Embodiments of a suitable expression vector or combination of expression vectors according to the present invention as well as suitable eukaryotic host cells are described in detail above; we refer to the above disclosure. Furthermore, besides stable transfection systems, also transient expression systems can be used, in particular when using the present selection system as a control for a RNAi experiment.

Also the RNAi inducing compound(s) can be introduced by the respective methods into the eukaryotic host cell. The introduction of the RNAi inducing compound(s) and the expression vector can be performed in parallel and thus at the same time or sequentially. They can also be transfected in form of a mixture.

According to one embodiment, the knock-down of the gene essential for cell survival endogenously expressed by the eukaryotic host cell induces or promotes cell death, in particular apoptosis. As is outlined above, the apoptotic phenotype has the advantage that non-transfected cells are efficiently eliminated from the cell population and furthermore, the apoptotic phenotype can be easily visually determined due to the altered morphology of the eukaryotic host cells. In order to quickly obtain the respective apoptotic phenotype and/or to efficiently induce cell death it is within the scope of the present invention to use at least a multiple selection strategy targeting at least two genes essential for cell survival endogenously expressed by the host cells as is described above.

According to a further embodiment, the knock-down of the gene essential for cell survival disturbs the cell cycle and thereby inhibits the growth of untransfected eukaryotic host cells.

According to a preferred embodiment, the gene essential for cell survival is selected from the following group:

| **Gene** | **Abbreviation** | **Function** |
|---|---|---|
| Ubiquitin | **ubb** | See below |
| Polokinase 1 | **Plk1** | See below |
| Topoisomerase 1 | **Top1** | unwinding of DNA double helix |
| Integrin beta1 | **ITGB1** | |
| RAD51 homolog (RecA homolog, E. coli) | **RAD 51** | involved in the homologous recombination and repair of DNA |
| Exportin 5 | **XPO5** | mediates the transport of proteins and other cargo between the nuclear and cytoplasmic compartments |
| Exportin 7 | **XPO7** | mediates the transport of proteins and other cargo between the nuclear and cytoplasmic compartments |
| RAN binding protein 17 | **RANBP17** | RAN-binding protein-17 is a member of the importin-beta superfamily of nuclear transport receptors |
| importin 11 | **IPO11** | mediates nucleocytoplasmic transport of protein and RNA cargoes |
| importin 13 | **IPO13** | mediates nucleocytoplasmic transport of protein and RNA cargoes |
| eukaryotic translation elongation factor 1 alpha 1 | **EEF1A1** | alpha subunit of the elongation factor-1 complex responsible for the enzymatic delivery of aminoacyl tRNAs to the ribosome |
| eukaryotic translation elongation factor 1 alpha 2 | **EEF1A2** | alpha subunit of the elongation factor-2 complex responsible for the enzymatic delivery of aminoacyl tRNAs to the ribosom |
| eukaryotic translation elongation factor 1 epsilon 1 | **EEF1E1** | In the cytoplasm, the encoded protein is an auxiliary component of the macromolecular aminoacyl-tRNA synthase complex |

Ubiquitin is a small protein that occurs in all eukaryotic cells. It performs a myriad of functions through conjugation to a large range of target proteins. A variety of different modifications can occur. The ubiquitin protein is highly conserved among eukaryotic species. A protein is marked with ubiquitin (ubiquitylation or ubiquitination) by a series of steps. Following addition of a single ubiquitin moiety to a protein substrate (monoubiquitination), further ubiquitin molecules can be added to the first, yielding a polyubiquitin chain. The ubiquitination system functions in a wide variety of central cellular processes, including apoptosis, cell cycle and division, DNA transcription and repair and differentiation and development. A knock-down of the Ubiquitin gene, in particular Ubiquitin B induces apoptosis in the host cells.

Preferred target sequences for Ubiquitin include but are not limited to the following target sequences:

| | |
|---|---|
| TAAAGGTTTCGTTGCATGGTA | SEQ. ID. No 1 |
| TCAGTAATAGCTGAACCTGTT | SEQ. ID. No 2 |
| GTGCCCAGTGATGGCATTACT | SEQ. ID. No 3 |
| AAGTTTAGAAATTACAAGTTT | SEQ. ID. No 4 |
| TTCAGTCATGGCATTCGCAGT | SEQ. ID. No 5 |
| TACTCTGCACTATAGCCATTT | SEQ. ID. No 6 |
| TTCAGTAATAGCTGAACCTGT | SEQ. ID. No 7 |
| TAATAGCTGAACCTGTTCAAA | SEQ. ID. No 8 |
| AAATGTTAATAAAGGTTTCGT | SEQ. ID. No 9 |
| GTTAATAAAGGTTTCGTTGCA | SEQ. ID. No 10 |
| TTAATAAAGGTTTCGTTGCAT | SEQ. ID. No 11 |
| GTAATAGCTGAACCTGTTCAA | SEQ. ID. No 12 |

Appropriate RNAi inducing compounds as defined herein such as siNAs which bind the corresponding mRNA transcript can be designed based upon the selected target sequence according to methods which are well-known and also well established in the prior art. Targeting of the corresponding transcripts by the RNAi inducing compounds results in efficient gene silencing. The described target sequences are located in the 3' UTR of the transcript of the endogenously expressed gene.

A further example for a gene involved in a central metabolic pathway is the plk1 gene (polokinase 1). Plk1 is an enzyme that catalyzes the chemical reaction of ATP and a protein to ADP + a phosphoprotein. Thus, the two substrates of this enzyme are ATP and a protein, whereas its two products are ADP and phosphoprotein. This enzyme belongs to the family of transferases, specifically those transferring a phosphate group to the sidechain oxygen atom of serine or threonine residues in proteins (protein-serine/threonine kinases). This enzyme participates in particular in several metabolic pathways, thereunder the cell cycle. A knock-down of the plk1 gene also induces apoptosis in the host cells.

In case a combination of RNAi inducing compounds as defined herein is used for selection which target different genes essential for cell survival, said RNAi inducing compounds can be transfected into the cells either at the same time, for example by using a transfection composition comprising both (in case two RNAi inducing compounds are used) RNAi inducing compounds or by sequentially introducing the RNAi inducing compounds into the cells. It is also within the scope of the present invention to use further RNAi inducing compounds targeting different genes, e.g. target genes under evaluation in an RNAi experiment (see below). Respective experimental RNAi inducing compounds can also be transfected either together or separately with the RNAi inducing compounds used for selecting the eukaryotic host cells that have incorporated the expression vector. As is outlined above, also the expression vector according to the present invention can be co-transfected with the RNAi inducing compound(s).

The degree of apoptosis/cell death induced by the RNAi inducing compound or combination of RNAi inducing compounds used for selection is preferably between 10% to 100%, 25% to 100%, 50% to 100% or 75% to 100%.

According to one embodiment, the RNAi inducing compound and/or the respective combination used for selection can be efficiently used for transfection in a concentration selected from the group consisting of at least 5 nM, at least 10 nM, at least 25 nM and at least 50 nM.

The selection method according to the present invention may additionally comprise a step of selecting at least one eukaryotic host cell which comprises the introduced expression vector.

Cells obtained as a result of the selection procedure of the present invention will generally be isolated and may be enriched from non- selected cells of the original cell population. They can be isolated and cultured as individual cells. They can also be used in one or more additional rounds of selection, optionally for additional qualitative or quantitative analysis, or can be used e. g. in development of a cell line for protein production. According to one embodiment, an enriched population of producing eukaryotic host cells selected as described above is directly used as population for the production of the polypeptide of interest with a good yield.

Also provided is a method for producing a product of interest, comprising culturing a eukaryotic host cell selected according to the teachings of the present invention under conditions that allow for the expression of the product of interest.

Using the eukaryotic host cells selected according to the present invention for producing a product of interest, in particular a polypeptide, has the advantage that the product of interest can be produced with a good yield as an efficient and also stringent selection system is provided. Thus, the present invention also provides an improved method for producing a product of interest, in particular as the risk of altering or damaging the eukaryotic host cell due to the selection procedure is reduced by using the RNAi based approach according to the present invention. Suitable eukaryotic host cells and details of the selection procedure and the expression vector are described above; we refer to the above disclosure.

The expressed product of interest may be obtained by disrupting the eukaryotic host cells. The polypeptides may also be expressed, e.g. secreted into the culture medium and can be obtained therefrom. Also combinations of the respective methods are possible. Thereby, products, in particular polypeptides can be produced and obtained/isolated efficiently with high yield. The obtained product may also be subject to further processing steps such as e.g. purification and/or modification steps in order to produce the product of interest in the desired quality.

The method for producing the product of interest may comprise at least one of the following steps:
- isolating the product of interest from said cell culture medium and/or from said eukaryotic host cell; and/or
- processing the isolated product of interest.

The product of interest, for example a polypeptide, produced in accordance with the present invention may be recovered, further purified, isolated and/or modified by methods known in the art. For example, the product may be recovered from the nutrient medium by conventional procedures including, but not limited to, centrifugation, filtration, ultra-filtration, extraction or precipitation. Purification may be performed by a variety of procedures known in the art including, but not limited to, chromatography (e.g. ion exchange, affinity, hydrophobic, chromatofocusing, and size exclusion), electrophoretic procedures (e.g., preparative isoelectric focusing), differential solubility (e.g. ammonium sulfate precipitation) or extraction.

The product of interest can be any biological product capable of being produced by transcription, translation or any other event of expression of the genetic information encoded by said polynucleotide. In this respect, the product will be an expression product. The product of interest may be selected from the group consisting of polypeptides, nucleic acids, in particular RNA. The product can be a pharmaceutically or therapeutically active compound, or a research tool to be utilized in assays and the like. In a particularly preferred embodiment, the product is a polypeptide, preferably a pharmaceutically or therapeutically active polypeptide, or a research tool to be utilized in diagnostic or other assays and the like. A polypeptide is accordingly not limited to any particular protein or group of proteins, but may on the contrary be any protein, of any size, function or origin, which one desires to select and/or express by the methods described herein. Accordingly, several different polypeptides of interest may be expressed/produced. The term polypeptide refers to a molecule comprising a polymer of amino acids linked together by a peptide bond(s). Polypeptides include polypeptides of any length, including proteins (e.g. having more than 50 amino acids) and peptides (e.g. 2 - 49 amino acids). Polypeptides include proteins and/or peptides of any activity or bioactivity, including e.g. bioactive polypeptides such as enzymatic proteins or peptides (e.g. proteases, kinases, phosphatases), receptor proteins or peptides, transporter proteins or peptides, bactericidal and/or endotoxin-binding proteins, structural proteins or peptides, immune polypeptides, toxins, antibiotics, hormones, growth factors, vaccines or the like. Said polypeptide may be selected from the group consisting of peptide hormones, interleukins, tissue plasminogen activators, cytokines, immunoglobulins, in particular antibodies or functional antibody fragments or variants thereof. In a most preferred embodiment the polypeptide is an immunoglobulin molecule or antibody, or a functional variant thereof, for example a chimeric, or a partly or totally humanized antibody. Such an antibody can be a diagnostic antibody, or a pharmaceutically or therapeutically active antibody.

Also provided is a product obtained by a method according to the present invention as defined above and in the claims. Said product is preferably a polypeptide.

Also provided is a method for performing an RNAi experiment, comprising the following steps:
a) introducing an expression vector into a eukaryotic host cell which endogenously expresses a gene essential for cell survival, wherein said expression vector comprises at least
   i) a recombinant polynucleotide encoding a product which corresponds to and/or is a functional variant or functional equivalent of the product of a gene essential for cell survival that is endogenously expressed by said eukaryotic host cell;
   ii) a recombinant polynucleotide encoding a product which corresponds to and/or is a variant or equivalent of the product of a target gene of interest expressed by said eukaryotic host cell;
b) introducing into said eukaryotic host cell a RNAi inducing compound targeting the expression of said target gene of interest expressed by said eukaryotic host cell, wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound;
c) culturing the eukaryotic host cells under selective conditions, wherein the selective conditions comprise at least the introduction of at least one RNAi inducing compound, wherein said RNAi inducing compound targets at least the gene essential for cell survival that is endogenously expressed by said eukaryotic host cell and wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

The described RNAi experiment provides a valuable control for RNAi analyses. Basically, an RNAi rescue experiment is performed. In order to ensure that the eukaryotic host cells of the control (the RNAi rescue experiment) have incorporated the expression vector used for the rescue experiment and therefore may provide a reliable result, basically the selection process according to the present invention is performed. According to the teaching of this embodiment, the expression vector at least comprises a recombinant polynucleotide that is used for selecting the eukaryotic host cells that have incorporated the expression vector, namely the restoration polynucleotide. Said restoration polynucleotide encodes a product which corresponds to and/or is a functional variant or functional equivalent of a gene essential for cell survival that is expressed by the eukaryotic host cell. As is outlined above, under selective culturing conditions using a selective RNAi inducing compound that targets said gene essential for cell survival, the expression of the restoration polynucleotide allows eukaryotic host cells that have incorporated the expression vector to survive under selective culturing conditions. Host cells that have not incorporated the expression vector die under selection conditions (see above).

Furthermore, the expression vector comprises a recombinant polynucleotide which encodes a product corresponding to and/or which is a functional variant or functional equivalent of a target gene of interest that is also expressed by the eukaryotic host cell. This polynucleotide is an example of a polynucleotide of interest and is herein also referred to as the rescue polynucleotide. During an RNAi experiment, a target gene is knocked down by the use of an experimental RNAi inducing compound such as a siRNA in order to analyse the role/function of the target gene by, for example, analysing the phenotype of the eukaryotic host cell that is obtained due to the knock down. In order to analyse the specificity of the used experimental RNAi inducing compound for the target gene of interest, it is important to analyse whether the original phenotype can be restored by introducing said recombinant rescue polynucleotide encoding a product which corresponds to and/or is a functional variant or functional equivalent of said gene of interest that is targeted by the experimental RNAi inducing compound. Said rescue polynucleotide is also comprised in the expression vector according to the teachings of the present invention. By applying the RNAi inducing compound used for selection as well as the experimental RNAi inducing compound used for targeting the gene of interest, a reliable RNAi rescue experiment can be performed which ensures that the observed rescued phenotype is attributable to eukaryotic host cells which have incorporated the expression vector and accordingly the recombinant polynucleotide encoding a restoration product for the target gene of interest that is analysed in the RNAi experiment.

Therefore, a valuable control is provided by the teachings of the present invention.

At least two or all of steps a, b and c may be performed in parallel or sequentially. The expression vector and/or the RNAi inducing compounds may also be introduced as a mixture.

In order to select eukaryotic host cells which have incorporated the expression vector according to the present invention and accordingly the rescue vector, preferably the selection method as is described in detail above is performed. Details regarding said method, the expression vector and its elements, the RNAi inducing compounds and the selective culturing conditions are described in detail above. We refer to the above disclosure which also applies here.

Also provided is a kit, wherein said kit comprises at least:
a) an expression vector to be introduced into a eukaryotic host cell which comprises
   (i) an expression cassette comprising a recombinant polynucleotide encoding a product which corresponds to and/or is a functional variant or functional equivalent of a gene essential for cell survival that is endogenously expressed by said eukaryotic host cell; and
   (ii) a further expression cassette comprising a multiple cloning site for inserting a recombinant polynucleotide of interest;
b) an RNAi inducing compound targeting said gene essential for cell survival that is endogenously expressed by said eukaryotic host cell, wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

The described kit components allow the selection of eukaryotic host cells which have incorporated the described expression vector. A respective kit can, for example, be used for expressing a product of interest and in particular for performing RNAi experiments. The described expression vector is designed to be able to comprise /incorporate a recombinant polynucleotide for example encoding the product of interest that is supposed to be expressed/produced and/or encoding a product which corresponds to and/or is a functional variant or functional equivalent of a target gene that is analysed in an RNAi experiment (a rescue polynucleotide). Possible applications/uses of a respective kit for expressing a product of interest and/or for an RNAi rescue experiment are described in detail above and also below in the examples. Please refer to the respective disclosure.

The expression vector comprises an expression cassette for inserting a further recombinant polynucleotide. Said recombinant polynucleotide may e.g. encode a product of interest, e. g. a product which corresponds to and/or is a functional variant or functional equivalent of a target gene that is analysed in an RNAi experiment. Herein, we also refer in this respect to the polynucleotide of interest. Therefore, the expression vector according to the present invention comprises an insertion site for inserting a polynucleotide of interest but not yet comprising the polynucleotide of interest. A respective "empty" expression vector can be used by the customer for inserting the polynucleotide of interest (e.g. the product to be expressed and/or the product for restoring the phenotype in case of an RNAi rescue experiment), thereby obtaining a ready to use expression vector that can used for transfection.

The incorporation of the recombinant polynucleotide of interest can be achieved by using appropriate cloning methods, for example by using restriction enzymes in order to insert the polynucleotide encoding the product of interest. For this purpose the expression vector comprises a multiple cloning site (MCS) which can e.g. be used in all reading frames. A respective multiple cloning site is an insertion site that is located within an expression cassette. A respective "empty" expression vector provides a useful tool e.g. for expressing different products of interests as the expression vector can be easily adapted to the intended use by inserting the polynucleotide encoding the desired product of interest.

Further details of the expression vector and the RNAi inducing compounds are described in detail above. We refer to the above disclosure.

### FIGURES

The following figures serve to illustrate the present invention without in any way limiting the scope thereof. In particular, the figures relate to preferred embodiments of the present invention:

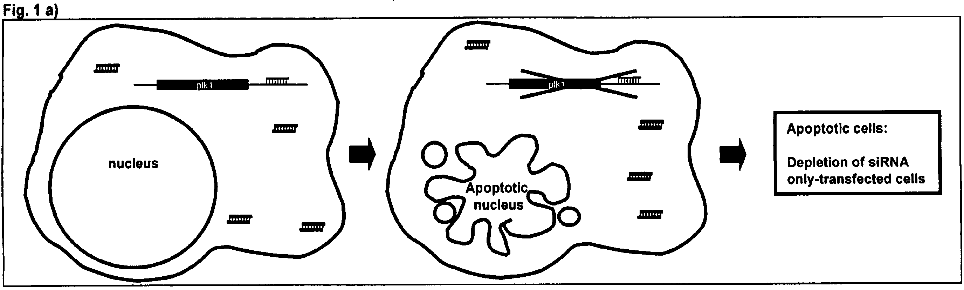

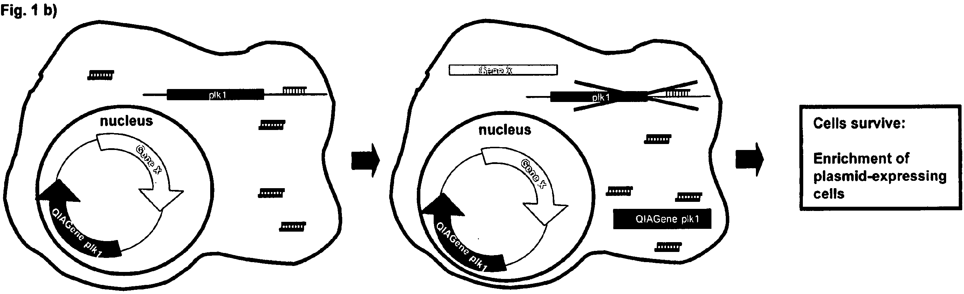

**Fig. 1** shows the embodiment wherein the method according to the present invention is used in a method to produce a polypeptide of interest in order to select host cells which have incorporated the expression vector and thus express a product of interest, e.g. a polypeptide.
**Fig. 1a** shows the scenario wherein the host cells have not incorporated the expression vector according to the present invention upon transfection. In the described embodiment, a selective RNAi inducing compound, here an siRNA targeting the plk1 gene is used as an example of a gene essential for cell survival. As can be seen in Fig. 1 a, the siRNA targets the 3' UTR of the plk1 transcript thereby efficiently inducing silencing of the endogenously expressed plk1 gene. Due to the RNAi induced knock-down of the plk1 gene, host cells that did not incorporate the expression vector, but only incorporated the siRNA directed against the plk1 transcript, undergo apoptosis and are therefore eliminated from the culture.
**Fig. 1 b** shows the scenario, wherein the host cells have successfully incorporated the expression vector according to the present invention. According to the shown embodiment, the vector is located in the nucleus but it could also be located in the cytoplasm. The expression vector comprises a restoration polynucleotide which encodes a product which corresponds to and/or is a functional variant or functional equivalent of the plk1 gene and hence a gene essential for cell survival that is endogenously expressed by the host cell. In Fig. 1b said restoration polynucleotide is referred to as QIAgene plk1. Furthermore, the expression vector comprises a recombinant polynucleotide encoding a product of interest that is according to the shown embodiment supposed to be expressed/produced by the host cell. Said polynucleotide is referred to as Gene X. The recombinant polynucleotides incorporated in the expression vector are expressed therefrom, the corresponding transcripts are indicated. During selection, the selective RNAi inducing compound, here an siRNA targeting plk1, targets the plk1 transcript that is derived from the plk1 gene endogenously expressed by the host cell thereby knocking-down the expression of the endogenous plk1 gene. However, said selective RNAi inducing compound does not target the QIAgene plk1 and thus the restoration polynucleotide/ transcript obtained from the expression vector. The restoration product is capable to restore the function of the knocked down endogenous plk1 gene and therefore allows the host cells that here incorporated the expression vector to survive the selection conditions.
   Accordingly, an efficient selection system is provided for selecting host cells that have incorporated the expression vector and according to the shown embodiment express/produce a product of interest, here a polypeptide from the Gene X. The QIAgene plk1 functions as an RNAi selectable marker gene.

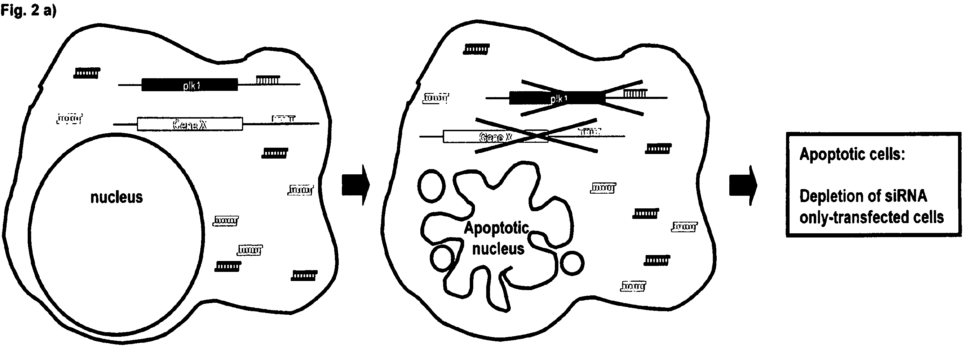

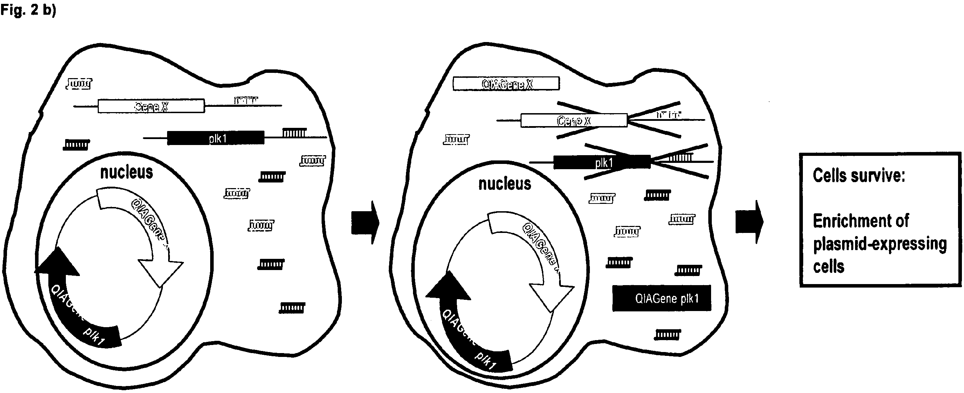

**Fig. 2** shows an embodiment wherein the method according to the present invention is used in an RNAi rescue experiment in order to select host cells which have incorporated the expression vector and thus express a rescue gene for restoring the wildtype phenotype. According to the shown embodiment, two different RNAi inducing compounds are used. One RNAi inducing compound (the experimental RNAi inducing compound) targets a target gene of interest expressed by the host cells, herein referred to as Gene X. The introduced experimental RNAi inducing compound, here an siRNA, targets the 3' UTR of the transcript of Gene X, thereby down-regulating its expression. The respective knock-down induces a phenotype that can be observed/analysed. In order to ensure that the observed phenotype is attributable to the knock-down of the endogenously expressed target gene and is not attributable to off-target effects, the shown RNAi resuce experiment that is usually performed in parallel as a control aims to restore the respective phenotype by introducing the rescue expression vector comprising a rescue polynucleotide QIAgene X.
In **Fig. 2a** the host cells did not incorporate the rescue expression vector and accordingly are unsuitable to allow the determination of a restored phenotype. Under the selective conditions, host cells that have not incorporated the rescue expression vector die and are accordingly eliminated, as a selective RNAi inducing compound (here also a siRNA) knock down a gene essential for cell survival that is endogenously expressed by the host cells, here plk1. Again, the respective siRNA targets the 3' UTR of plk1.
In **Fig. 2b** the host cells have successfully incorporated the rescue expression vector; in the shown embodiment again into the nucleus. Again, the rescue expression vector comprises the restoration polynucleotide encoding the product that corresponds to and/or is a functional variant or functional equivalent of the product of the gene essential for cell survival, here plk1. The respective restoration polynucleotide is referred to as QIAgene plk1. The expression of the QIAgene plk1 as a RNAi selectable marker gene allows the host cells to survive the selective growth conditions wherein a selective RNAi inducing compound targeting the expression of the endogenous plk1 gene is used (see above). Furthermore, the rescue expression vector comprises a QIAgene X which encodes a product which corresponds to and/or is a functional variant or functional equivalent of the product encoded by a target gene that is attacked by the experimental RNAi inducing compound under evaluation. The respective rescue polynucleotide of interest is expressed from the expression vector and can therefore restore compensate the expression of the knocked down endogenously expressed target gene X. Thereby, the knock-down of the endogenously expressed target gene X is restored due to the incorporation of the expression vector and it can be analysed in a respective successful RNAi rescue experiment, whether the RNAi inducing compound used for targeting the gene of interest X is specific for the respective target gene. In case the original phenotype is obtained, this is the case. In case one is not able to restore the respective wildtype phenotype by incorporating the expression vector, this is an indicator that the RNAi inducing compound used induces off-target effects and that the experimental phenotype observed upon knock-down of the expression of the gene is not specifically attributable to the knock-down of the respective gene.

Thereby, a very valuable and suitable RNAi rescue experiment/control is provided by the teachings of the present invention.

## Claims

1. A method for selecting a eukaryotic host cell comprising an introduced expression vector, comprising the following steps:
a) Providing a population of eukaryotic host cells, wherein said host cells endogenously express at least one gene essential for cell survival;
b) Introducing an expression vector into said eukaryotic host cells, wherein said expression vector comprises at least one recombinant polynucleotide encoding a product which corresponds to and/or is a functional variant or functional equivalent of the product encoded by said gene essential for cell survival that is endogenously expressed by said eukaryotic host cells;
c) Culturing the eukaryotic host cells under selective conditions, wherein the selective conditions comprise at least the introduction of at least one RNAi inducing compound into said eukaryotic host cells, wherein said RNAi inducing compound targets at least said gene essential for cell survival that is endogenously expressed by said eukaryotic host cells and wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

2. The method according to claim 1, wherein said method has at least one of the following characteristics:
a) at least one eukaryotic host cell is selected that survives the selective growth conditions; and/or
b) eukaryotic host cells that did not incorporate efficiently the expression vector do not survive under the selective culture conditions; and/or
c) the selective culture conditions comprise the introduction of at least one further RNAi inducing compound which targets a second gene essential for cell survival that is endogenously expressed by said eukaryotic host cell.

3. The method according to claim 1 or 2, wherein said expression vector has at least one of the following characteristics:
a) said recombinant polynucleotide encoding a product which corresponds to and/or is a functional variant or functional equivalent of the product encoded by said gene essential for cell survival that is endogenously expressed by said host cells is an RNAi selectable marker gene, wherein an RNAi selectable marker gene is a gene which provides resistance to an RNAi inducing compound introduced into the eukaryotic host cell and which allows the selection of eukaryotic host cells that have incorporated the expression vector under the selection conditions; and/or
b) it comprises a further recombinant polynucleotide encoding a product which corresponds to and/or is a functional variant or functional equivalent of the product of said second gene essential for cell survival that is endogenously expressed by said eukaryotic host cell; and/or
c) it comprises a further recombinant polynucleotide encoding a product of interest; and/or
d) at least one recombinant polynucleotide is encompassed in an expression cassette; and/or
e) at least one recombinant polynucleotide is encompassed in an expression cassette, wherein said expression cassette comprises at least one of the following elements:
i. a promoter and/or enhancer sequence functional in said eukaryotic host cells;
ii. a transcriptional termination signal;
iii. a poly A site;
iv. an intron;
v. a secretory leader sequence;
and/or
f) it comprises at least one origin of replication; and/or
g) it comprises at least one additional selectable marker gene; and/or
h) said recombinant polynucleotide encoding a product which corresponds to and/or is a functional variant or functional equivalent of the product encoded by said gene essential for cell survival that is endogenously expressed by said eukaryotic host cells is resistant to the RNAi inducing compound used for selection.

4. The method according to one of the claims 1 to 3, wherein said RNAi inducing compound has one or more of the following characteristics:
a) said RNAi inducing compound does not target the transcript of said recombinant polynucleotide encoding a product which corresponds to and/or is a functional variant or functional equivalent of the product encoded by said gene essential for cell survival that is endogenously expressed by said host cells; and/or
b) said RNAi inducing compound targets an untranslated region of said gene essential for cell survival that is endogenously expressed by said host cells; and/or
c) said RNAi inducing compound targets an untranslated region of said gene essential for cell survival that is endogenously expressed by said host cell which is not present in the recombinant polynucleotide encoding a product which corresponds to and/or is a functional variant or functional equivalent of the product encoded by said gene essential for cell survival that is endogenously expressed by said host cells; and/or
d) said RNAi inducing compound is selected from the group consisting of
i. it is a siRNA, miRNA or a shRNA; and/or
ii. it is an siRNA double-stranded molecule of preferably 18 to 30 nt; and/or
iii. it is expressed by a vector.

5. The method according to any one of claims 1 to 4, wherein said eukaryotic host cells have one of the following characteristics:
a) it is an insect cell;
b) it is a mammalian cell.

6. The method according to any one of claims 1 to 5, wherein said gene essential for cell survival endogenously expressed by said host cell has at least one of the following characteristics:
a) It's knock-down induces or promotes apoptosis; and/or
b) It's knock-down disturbs the cell cycle; and/or
c) Said gene is selected from the group consisting of
| |
|---|
| Ubiquitin |
| Polokinase 1 |
| Topoisomerase 1 |
| Integrin beta1 |
| RAD51 homolog (RecA homolog, E. coli) |
| Exportin 5 |
| Exportin 7 |
| RAN binding protein 17 |
| importin 11 |
| importin 13 |
| eukaryotic translation elongation factor 1 alpha 1 |
| eukaryotic translation elongation factor 1 alpha 2 |
| eukaryotic translation elongation factor 1 epsilon 1 |

7. A method for producing a product of interest, comprising culturing a eukaryotic host cell selected according to any one of claims 1 to 6 under conditions that allow for the expression of the product of interest.

8. The method according to claim 7, comprising at least one of the following steps:
- isolating the product of interest from said cell culture medium and/or from said eukaryotic host cell; and/or
- processing the isolated product of interest.

9. A method for performing an RNAi experiment, comprising the following steps:
a) introducing an expression vector into a eukaryotic host cell which endogenously expresses a gene essential for cell survival, wherein said expression vector comprises at least
i) a recombinant polynucleotide encoding a product which corresponds to and/or is a functional variant or functional equivalent of the product of a gene essential for cell survival that is endogenously expressed by said eukaryotic host cell;
ii) a recombinant polynucleotide encoding a product which corresponds to and/or is a variant or equivalent of the product of a target gene of interest expressed by said eukaryotic host cell;
b) introducing into said host cell a RNAi inducing compound targeting the expression of said target gene of interest expressed by said eukaryotic host cell, wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound;
c) culturing the eukaryotic host cells under selective conditions, wherein the selective conditions comprise at least the introduction of at least one RNAi inducing compound, wherein said RNAi inducing compound targets at least the gene essential for cell survival that is endogenously expressed by said eukaryotic host cell and wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

10. The method according to claim 9, wherein at least two or all of steps a, b and c are performed in parallel or sequentially.

11. The method according to claim 9 or 10, wherein the selection method according to at least one of the claims 2 to 6 is performed.

12. A kit comprising at least:
a) an expression vector to be introduced into a eukaryotic host cell which comprises
(i) an expression cassette comprising a recombinant polynucleotide encoding a product which corresponds to and/or is a functional variant or functional equivalent of a gene essential for cell survival that is endogenously expressed by said eukaryotic host cell;
and
a further expression cassette comprising a multiple cloning site for inserting a recombinant polynucleotide of interest;
b) an RNAi inducing compound targeting said gene essential for cell survival that is endogenously expressed by said eukaryotic host cell, wherein said RNAi inducing compound is selected from short interfering nucleic acids (siNA), short interfering RNA (siRNA), microRNA (miRNA) and short hairpin RNAs (shRNA) as well as precursors thereof which are processed in the cell to the actual RNAi inducing compound.

13. The kit according to claim 12, wherein said expression vector has at least one of the following characteristics:
a) said recombinant polynucleotide encoding a product which corresponds to and/or is a functional variant or functional equivalent of the product encoded by said gene essential for cell survival that is endogenously expressed by said eukaryotic host cells is an RNAi selectable marker gene, wherein a RNAi selectable marker gene is a gene which provides resistance to an RNAi inducing compound introduced into the eukaryotic host cell and which allows the selection of eukaryotic host cells that have incorporated the expression vector under the selection conditions; and/or
b) it comprises a further recombinant polynucleotide encoding a product which corresponds to and/or is a functional variant or functional equivalent of the product of said second gene essential for cell survival that is endogenously expressed by said eukaryotic host cell; and/or
c) at least one recombinant polynucleotide is encompassed in the expression cassette; and/or
d) at least one recombinant polynucleotide is encompassed in the expression cassette, wherein said expression cassette comprises at least one of the following elements:
i. a promoter and/or enhancer sequence functional in said eukaryotic host cells;
ii. a transcriptional termination signal;
iii. a poly A site;
iv. an intron;
v. a secretory leader sequence;
and/or
e) it comprises at least one origin of replication; and/or
f) it comprises at least one additional selection marker gene; and/or
g) said recombinant polynucleotide encoding a product which corresponds to and/or is a functional variant or functional equivalent of the product encoded by said gene essential for cell survival that is endogenously expressed by said eukaryotic host cells is resistant to the RNAi inducing compound used for selection.

14. The kit according to claim 12 or 13, wherein said RNAi inducing compound has one or more of the following characteristics:
a) said RNAi inducing compound does not target the transcript of said recombinant polynucleotide encoding a product which corresponds to and/or is a functional variant or functional equivalent of the product encoded by said gene essential for cell survival that is endogenously expressed by said eukaryotic host cells; and/or
b) said RNAi inducing compound targets an untranslated region of said gene essential for cell survival that is endogenously expressed by said eukaryotic host cells; and/or
c) said RNAi inducing compound targets an untranslated region of said gene essential for cell survival that is endogenously expressed by said eukaryotic host cells that is not present in the recombinant polynucleotide encoding a product which corresponds to and/or is a functional variant or functional equivalent of the product encoded by said gene essential for cell survival that is endogenously expressed by said eukaryotic host cells; and/or
d) said gene essential for cell survival endogenously expressed by said eukaryotic host cell has at least one of the following characteristics:
i. Its knock-down induces or promotes apoptosis; and/or
ii. Its knock-down disturbs the cell cycle; and/or
iii. said gene is selected from the group consisting of
| |
|---|
| Ubiquitin |
| Polokinase 1 |
| Topoisomerase 1 |
| Inteqrin beta1 |
| RAD51 homolog (RecA homolog, E. coli) |
| Exportin 5 |
| Exportin 7 |
| RAN binding protein 17 |
| importin 11 |
| importin 13 |
| eukaryotic translation elongation factor 1 alpha 1 |
| eukaryotic translation elongation factor 1 alpha 2 |
| eukaryotic translation elongation factor 1 epsilon 1 |
and/or
e) said RNAi inducing compound is selected from the group consisting of
i. it is a siRNA, miRNA or a shRNA; and/or
ii. it is an siRNA double-stranded molecule of preferably 18 to 30 nt; and/or
iii. it is expressed by a vector.

15. Use of a kit according to one or more of claims 12 to 14 for expressing a product of interest or for performing RNAi experiments.

## Patentansprüche

1. Verfahren zur Selektion einer einen eingeführten Expressionsvektor enthaltenden eukaryontischen Wirtszelle, umfassend die folgenden Schritte:
a) Bereitstellen einer Population eukaryontischer Wirtszellen, wobei die Wirtszellen endogen wenigstens ein für das Überleben der Zelle essentielles Gen exprimieren;
b) Einführen eines Expressionsvektors in die eukaryontischen Wirtszellen, wobei der Expressionsvektor wenigstens ein rekombinantes Polynukleotid aufweist, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den eukaryontischen Wirtszellen exprimiert wird;
c) Kultivieren der eukaryontischen Wirtszellen unter selektiven Bedingungen, wobei die selektiven Bedingungen wenigstens die Einführung wenigstens einer RNAi induzierenden Verbindung in die eukaryontischen Wirtszellen umfassen, wobei die RNAi induzierende Verbindung wenigstens auf das für das Überleben der Zellen essentielle Gen, das endogen von den eukaryontischen Wirtszellen exprimiert wird, abzielt und wobei die RNAi induzierende Verbindung ausgewählt ist aus siNA (Short Interfering Nucleic Acids), siRNA (Short Interfering RNA), miRNA (micro RNA) und shRNA (Short Hairpin RNA) sowie Vorstufen davon, die in der Zelle zur eigentlichen RNAi induzierenden Verbindung prozessiert werden.

2. Verfahren nach Anspruch 1, wobei das Verfahren wenigstens eines der folgenden Charakteristika aufweist:
a) es wird wenigstens eine eukaryontische Wirtszelle selektioniert, die die selektiven Wachstumsbedingungen überlebt; und/oder
b) eukaryontische Wirtszellen, bei denen der Expressionsvektor nicht effizient eingebaut wurde, überleben unter den selektiven Kulturbedingungen nicht; und/oder
c) die selektiven Kulturbedingungen umfassen die Einführung wenigstens einer weiteren RNAi induzierenden Verbindung, die auf ein zweites für das Überleben der Zellen essentielles Gen, das endogen von der eukaryontischen Wirtszelle exprimiert wird, abzielt.

3. Verfahren nach Anspruch 1 oder 2, wobei der Expressionsvektor wenigstens eines der folgenden Charakteristika aufweist:
a) bei dem rekombinanten Polynukleotid, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den Wirtszellen exprimiert wird, handelt es sich um ein RNAi-selektionierbares Marker-Gen, wobei ein RNAi-selektionierbaren Marker-Gen ein Gen ist, das eine Resistenz gegen eine in die eukaryontische Wirtszelle eingeführte RNAi induzierende Verbindung vermittelt und das unter den Selektionsbedingungen die Selektion eukaryontischer Wirtszellen, bei denen der Expressionsvektor eingebaut wurde, gestattet; und/oder
b) er umfasst ein weiteres rekombinantes Polynukleotid, das für ein Produkt codiert, welches dem Produkt entspricht und/oder eine funktionelle Variante oder ein funktionelles Äquivalent des Produktes ist, das durch das für das Überleben der Zelle essentielle zweite Gen codiert wird, das endogen von der eukaryontischen Wirtszelle exprimiert wird; und/oder
c) er umfasst ein weiteres rekombinantes Polynukleotid, das für ein Produkt von Interesse codiert; und/oder
d) wenigstens ein rekombinantes Polynukleotid ist in einer Expressionskassette beinhaltet; und/oder
e) wenigstens ein rekombinantes Polynukleotid ist in einer Expressionskassette beinhaltet, wobei die Expressionskassette wenigstens eines der folgenden Elemente aufweist:
i. eine in den eukaryontischen Wirtszellen funktionsfähige Promotor- und/oder Enhancer-Sequenz;
ii: ein Transkriptionsterminationssignal;
iii. eine Poly-A-Stelle;
iv: ein Intron;
v. eine sekretorische Leitsequenz;
und/oder
f) er umfasst wenigstens einen Replikationsursprung; und/oder
g) er umfasst wenigstens ein zusätzliches selektionierbares Marker-Gen; und/oder
h) das rekombinante Polynukleotid, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den eukaryontischen Wirtszellen exprimiert wird, ist gegen die zur Selektion verwendete RNAi induzierende Verbindung resistent.

4. Verfahren nach einem der Ansprüche 1 bis 3, wobei die RNAi induzierende Verbindung eines oder mehrere der folgenden Charakteristika aufweist:
a) die RNAi induzierende Verbindung zielt nicht auf das Transkript des rekombinanten Polynukleotids, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den Wirtszellen exprimiert wird; und/oder
b) die RNAi induzierende Verbindung zielt auf einen nicht translatierten Bereich des für das Überleben der Zellen essentiellen Gens, das endogen von den Wirtszellen exprimiert wird; und/oder
c) die RNAi induzierende Verbindung zielt auf einen nicht translatierten Bereich des für das Überleben der Zellen essentiellen Gens, das endogen von der Wirtszelle exprimiert wird, ab, der in dem rekombinanten Polynukleotid, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den Wirtszellen exprimiert wird, nicht vorhanden ist; und/oder
d) die RNAi induzierende Verbindung ist aus der folgenden Gruppe ausgewählt:
i. es handelt sich um eine siRNA, miRNA oder eine shRNA; und/oder
ii. es handelt sich um ein doppelsträngiges siRNA-Molekül mit vorzugsweise 18 bis 30 nt; und/oder
iii. sie wird von einem Vektor exprimiert.

5. Verfahren nach irgendeinem der Ansprüche 1 bis 4, wobei die eukaryontischen Wirtszellen eines der folgenden Charakteristika aufweisen:
a) es handelt sich um eine Insektenzelle;
b) es handelt sich um eine Säugerzelle.

6. Verfahren nach irgendeinem der Ansprüche 1 bis 5, wobei das endogen von der Wirtszelle exprimierte, für das Überleben der Zellen essentielle Gen wenigstens eines der folgenden Charakteristika aufweist:
a) sein Knock-down induziert oder fördert Apoptose; und/oder
b) sein Knock-down stört den Zellzyklus; und/oder
c) das Gen ist aus der folgenden Gruppe ausgewählt:
| |
|---|
| Ubiquitin |
| Polykinase 1 |
| Topoisomerase 1 |
| Inteqrin-beta 1 |
| RAD51-Homolog (RecA-Homolog, E. coli) |
| Exportin 5 |
| Exportin 7 |
| RAN-Bindungsprotein 17 |
| Importin 11 |
| Importin 13 |
| Eukaryontischer Translationselonqationsfaktor 1 alpha 1 |
| Eukaryontischer Translationselongationsfaktor 1 alpha 2 |
| Eukaryontischer Translationselongationsfaktor 1 epsilon 1 |

7. Verfahren zur Herstellung eines Produktes von Interesse, bei dem man eine gemäß irgendeinem der Ansprüche 1 bis 6 selektionierte eukaryontische Wirtszelle unter Bedingungen kultiviert, die die Expression des Produkts von Interesse gestatten.

8. Verfahren nach Anspruch 7, aufweisend wenigstens einen der folgenden Schritte:
- Isolieren des Produktes von Interesse aus dem Zellkulturmedium und/oder aus der eukaryontischen Wirtszelle; und/oder
- Verarbeiten des isolierten Produktes von Interesse.

9. Verfahren zur Durchführung eines RNAi-Experiments, aufweisend die folgenden Schritte:
a) Einführen eines Expressionsvektors in eine eukaryontische Wirtszelle, die endogen ein für das Überleben der Zellen essentielles Gen exprimiert, wobei der Expressionsvektor wenigstens aufweist
i) ein rekombinantes Polynukleotid, das für ein Produkt codiert, das dem Produkt entspricht und/oder eine funktionelle Variante oder ein funktionelles Äquivalent des Produktes ist, das durch das für das Überleben der Zelle essentielle Gen codiert wird, das endogen von der eukaryontischen Wirtszelle exprimiert wird;
ii) ein rekombinantes Polynukleotid, das für ein Produkt codiert, das dem Produkt eines Zielgens von Interesse entspricht und/oder eine Variante oder ein Äquivalent dieses Produktes ist, dass durch die eukaryontische Wirtszelle exprimiert wird;
b) Einführen in die Wirtszelle einer auf die Expression des von der eukaryontischen Wirtszelle exprimierten Zielgens von Interesse abzielenden RNAi induzierenden Verbindung, wobei die RNAi induzierende Verbindung ausgewählt ist aus siNA (Short Interfering Nucleic Acids), siRNA (Short Interfering RNA), miRNA (micro RNA) und shRNA (Short Hairpin RNA) sowie Vorstufen davon, die in der Zelle zur eigentlichen RNAi induzierenden Verbindung prozessiert werden;
c) Kultivieren der eukaryontischen Wirtszellen unter selektiven Bedingungen, wobei die selektiven Bedingungen wenigstens die Einführung wenigstens einer RNAi induzierenden Verbindung umfassen, wobei die RNAi induzierende Verbindung auf wenigstens das für das Überleben der Zelle essentielle Gen, das endogen von der eukaryontischen Wirtszelle exprimiert wird, abzielt und wobei die RNAi induzierende Verbindung ausgewählt ist aus siNA (Short Interfering Nucleic Acids), siRNA (Short Interfering RNA), miRNA (MicroRNA) und shRNA (Short Hairpin RNA) sowie Vorstufen davon, die in der Zelle zur eigentlichen RNAi induzierenden Verbindung prozessiert werden.

10. Verfahren nach Anspruch 9, wobei wenigstens zwei der oder alle Schritte a, b und c parallel oder nacheinander durchgeführt werden.

11. Verfahren nach Anspruch 9 oder 10, wobei das Selektionsverfahren nach wenigstens einem der Ansprüche 2 bis 6 durchgeführt wird.

12. Kit, aufweisend wenigstens:
a) einen in eine eukaryontische Wirtszelle einzuführenden Expressionsvektor, aufweisend:
(i) eine Expressionskassette, die ein rekombinantes Polynukleotid aufweist, das ein Produkt codiert, das dem Produkt entspricht und/oder eine funktionelle Variante oder ein funktionelles Äquivalent des Produktes ist, das durch das für das Überleben der Zelle essentielle Gen codiert wird, das endogen von der eukaryontischen Wirtszelle exprimiert wird;
und
(ii) eine weitere Expressionskassette, die eine Mehrfachklonierungsstelle zur Insertion eines rekombinanten Polynukleotids von Interesse aufweist;
b) eine RNAi induzierende Verbindung, die auf das für das Überleben der Zellen essentielle Gen, das endogen von der eukaryontischen Wirtszelle exprimiert wird, abzielt, wobei die RNAi induzierende Verbindung ausgewählt ist aus siNA (Short Interfering Nucleic Acids), siRNA (Short Interfering RNA), miRNA (MicroRNA) und shRNA (Short Hairpin RNA) ebenso wie Vorstufen davon, die in der Zelle zur eigentlichen RNAi induzierenden Verbindung prozessiert werden.

13. Kit nach Anspruch 12, wobei der Expressionsvektor wenigstens eines der folgenden Charakteristika aufweist:
a) bei dem rekombinanten Polynukleotid, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den Wirtszellen exprimiert wird, handelt es sich um ein RNAi-selektionierbares Marker-Gen, wobei ein RNAi-selektionierbaren Marker-Gen ein Gen ist, das eine Resistenz gegen eine in die eukaryontische Wirtszelle eingeführte RNAi induzierende Verbindung vermittelt und das unter den Selektionsbedingungen die Selektion eukaryontischer Wirtszellen, bei denen der Expressionsvektor eingebaut wurde, gestattet; und/oder
b) er umfasst ein weiteres rekombinantes Polynukleotid, das für ein Produkt codiert, welches dem Produkt entspricht und/oder eine funktionelle Variante oder ein funktionelles Äquivalent des Produktes ist, das durch das für das Überleben der Zelle essentielle zweite Gen codiert wird, das endogen von der eukaryontischen Wirtszelle exprimiert wird; und/oder
c) wenigstens ein rekombinantes Polynukleotid ist in der Expressionskassette beinhaltet; und/oder
d) wenigstens ein rekombinantes Polynukleotid ist in der Expressionskassette beinhaltet, wobei die Expressionskassette wenigstens eines der folgenden Elemente umfasst:
i. eine in den eukaryontischen Wirtszellen funktionsfähige Promotor- und/oder Enhancer-Sequenz;
ii. ein Transkriptionsterminationssignal;
iii. eine Poly-A-Stelle;
iv. ein Intron;
v. eine sekretorische Leitsequenz;
und/oder
e) er umfasst wenigstens einen Replikationsursprung; und/oder
f) er umfasst wenigstens ein zusätzliches selektionierbares Marker-Gen; und/oder
g) das rekombinante Polynukleotid, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den eukaryontischen Wirtszellen exprimiert wird, ist gegen die zur Selektion verwendete RNAi induzierende Verbindung resistent.

14. Kit nach Anspruch 12 oder 13, wobei die RNAi induzierende Verbindung eines oder mehrere der folgenden Charakteristika aufweist:
a) die RNAi induzierende Verbindung zielt nicht auf das Transkript des rekombinanten Polynukleotids, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den eukaryontischen Wirtszellen exprimiert wird; und/oder
b) die RNAi induzierende Verbindung zielt auf einen nicht translatierten Bereich des für das Überleben der Zellen essentiellen Gens, das endogen von den eukaryontischen Wirtszellen exprimiert wird, ab; und/oder
c) die RNAi induzierende Verbindung zielt auf einen nicht translatierten Bereich des für das Überleben der Zellen essentiellen Gens, das endogen von der Wirtszelle exprimiert wird, ab, der in dem rekombinanten Polynukleotid, das für ein Produkt codiert, welches dem Produkt entspricht und/oder eine funktionelle Variante oder ein funktionelles Äquivalent des Produktes ist, das durch das für das Überleben der Zelle essentielle Gen codiert wird, das endogen von den eukaryontischen Wirtszellen exprimiert wird, nicht vorhanden ist; und/oder
d) das endogen von der eukaryontischen Wirtszelle exprimierte für das Überleben der Zellen essentielle Gen weist wenigstens eines der folgenden Charakteristika auf:
i. sein Knock-down induziert oder fördert Apoptose; und/oder
ii. sein Knock-down stört den Zellzyklus; und/oder
iii. das Gen ist aus der folgenden Gruppe ausgewählt:
| |
|---|
| Ubiquitin |
| Polykinase 1 |
| Topoisomerase 1 |
| Integrin-beta 1 |
| RAD51-Homolog (RecA-Homolog, E. coli) |
| Exportin 5 |
| Exportin 7 |
| RAN-Bindungsprotein 17 |
| Importin 11 |
| Importin 13 |
| Eukaryontischer Translationselonqationsfaktor 1 alpha 1 |
| Eukaryontischer Translationselongationsfaktor 1 alpha 2 |
| Eukaryontischer Translationselonqationsfaktor 1 epsilon 1 |
und/oder
e) die RNAi induzierende Verbindung ist aus der folgenden Gruppe ausgewählt:
i. es handelt sich um eine siRNA, miRNA oder eine shRNA; und/oder
ii. es handelt sich um ein doppelsträngiges siRNA-Molekül mit vorzugsweise 18 bis 30 Nt; und/oder
iii. sie wird von einem Vektor exprimiert.

15. Verwendung eines Kits nach einem oder mehreren der Ansprüche 12 bis 14 zur Expression eines Produkts von Interesse oder zur Durchführung von RNAi-Experimenten.

## Revendications

1. Procédé de sélection d'une cellule hôte eucaryote comprenant un vecteur d'expression introduit, comprenant les étapes suivantes consistant à:
a) fournir une population de cellules hôtes eucaryotes, où lesdites cellules hôtes expriment de façon endogène au moins un gène essentiel à la survie cellulaire;
b) introduire un vecteur d'expression dans lesdites cellules hôtes eucaryotes, où ledit vecteur d'expression comprend au moins un polynucléotide recombinant codant pour un produit qui correspond à et/ou qui est un variant fonctionnel ou un équivalent fonctionnel du produit codé par ledit gène essentiel à la survie cellulaire qui est exprimé de façon endogène par lesdites cellules hôtes eucaryotes;
c) cultiver les cellules hôtes eucaryotes dans des conditions sélectives, où les conditions sélectives comprennent au moins l'introduction d'au moins un composé induisant un ARNi dans lesdites cellules hôtes eucaryotes, où ledit composé induisant un ARNi cible au moins ledit gène essentiel à la survie cellulaire qui est exprimé de façon endogène par lesdites cellules hôtes eucaryotes et où ledit composé induisant un ARNi est sélectionné parmi des petits acides nucléiques interférents (siNA), un petit ARN interférent (siRNA), un microARN (miRNA) et des petits ARN en épingle à cheveux (shRNA), ainsi que leurs précurseurs qui sont traités dans la cellule en composé réel induisant l'ARNi.

2. Procédé selon la revendication 1, dans lequel ledit procédé présente au moins une des caractéristiques suivantes:
a) on sélectionne au moins une cellule hôte eucaryote qui survit aux conditions de croissance sélectives; et/ou
b) les cellules hôtes eucaryotes qui n'incorporent pas de façon efficace le vecteur d'expression ne survivent pas dans les conditions de culture sélectives; et/ou
c) les conditions de culture sélectives comprennent l'introduction d'au moins un autre composé induisant l'ARNi qui cible un second gène essentiel à la survie cellulaire qui est exprimé de façon endogène par ladite cellule hôte eucaryote.

3. Procédé selon la revendication 1 ou 2, dans lequel ledit vecteur d'expression présente au moins une des caractéristiques suivantes:
a) ledit polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes est un gène de marqueur sélectionnable d'ARNi, où le gène de marqueur sélectionnable d'ARNi est un gène qui confère une résistance à un composé induisant l'ARNi introduit dans la cellule hôte eucaryote et qui permet la sélection de cellules hôtes eucaryotes qui ont incorporé le vecteur d'expression dans les conditions de sélection; et/ou
b) il comprend un autre polynucléotide recombinant codant pour un produit qui correspond à et/ou est un variant fonctionnel ou un équivalent fonctionnel du produit dudit second gène essentiel à la survie cellulaire qui est exprimé de façon endogène par ladite cellule hôte eucaryote; et/ou
c) il comprend un autre polynucléotide recombinant codant pour un produit intéressant; et/ou
d) au moins un polynucléotide recombinant est incorporé dans une cassette d'expression; et/ou
e) au moins un polynucléotide recombinant est incorporé dans une cassette d'expression, où ladite cassette d'expression comprend au moins un des éléments suivants:
i. un promoteur et/ou une séquence activatrice fonctionnelle dans lesdites cellules hôtes eucaryotes;
ii. un signal de terminaison transcriptionnelle;
iii. un site poly A;
iv. un intron;
v. une séquence leader sécrétoire;
et/ou
f) il comprend au moins une origine de réplication ; et/ou
g) il comprend au moins un gène de marqueur sélectionnable supplémentaire ; et/ou
h) ledit polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes eucaryotes est résistant au composé induisant l'ARNi utilisé pour la sélection.

4. Procédé selon l'une des revendications 1 à 3, dans lequel ledit composé induisant l'ARNi présente une ou plusieurs des caractéristiques suivantes:
a) ledit composé induisant l'ARNi ne cible pas le transcript dudit polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes eucaryotes; et/ou
b) ledit composé induisant l'ARNi cible une région non traduite dudit gène essentiel à la survie cellulaire qui est exprimé de façon endogène par lesdites cellules hôtes eucaryotes; et/ou
c) ledit composé induisant l'ARNi cible une région non traduite dudit gène essentiel à la survie cellulaire qui est exprimé de façon endogène par lesdites cellules hôtes eucaryotes qui n'est pas présente dans le polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes eucaryotes; et/ou
d) ledit composé induisant l'ARNi est sélectionné dans le groupe composé de:
i. c'est un siRNA, un miRNA ou un shRNA ; et/ou
ii. c'est une molécule double brin de siRNA comportant de préférence 18 à 30 nt ; et/ou
iii. il est exprimé par un vecteur.

5. Procédé selon l'une quelconque des revendications 1 à 4, dans lequel lesdites cellules hôtes eucaryotes présentent une des caractéristiques suivantes:
a) c'est une cellule d'insecte ;
b) c'est une cellule de mammifère.

6. Procédé selon l'une quelconque des revendications 1 à 5, dans lequel ledit gène essentiel à la survie cellulaire exprimé de façon endogène par ladite cellule hôte présente au moins l'une des caractéristiques suivantes:
a) son knock-down induit ou favorise l'apoptose ; et/ou
b) son knock-down perturbe le cycle cellulaire ; et/ou
c) ledit gène est sélectionné dans le groupe composé de :
| |
|---|
| Ubiquitine |
| Polokinase 1 |
| Topoisomérase 1 |
| Intégrine bêta 1 |
| Homoloque de RAD51 (homol. RecA, E. coli) |
| Exportine 5 |
| Exportine 7 |
| Protéine de liaison RAN 17 |
| Importine 11 |
| Importine 13 |
| Facteur 1 alpha 1 d'élongation de traduction eucaryote |
| Facteur 1 alpha 2 d'élongation de traduction eucaryote |
| Facteur 1 epsilon 1 d'élongation de traduction eucaryote |

7. Procédé de production d'un produit intéressant, comprenant la culture d'une cellule hôte eucaryote sélectionnée selon l'une quelconque des revendications 1 à 6 dans des conditions qui permettent l'expression du produit présentant un intérêt.

8. Procédé selon la revendication 7, comprenant au moins l'une des étapes suivantes:
- isolement du produit intéressant depuis ledit milieu de culture cellulaire et/ou depuis ladite cellule hôte eucaryote ; et/ou
- traitement du produit intéressant isolé.

9. Procédé de réalisation d'une expérience sur l'ARNi, comprenant les étapes suivantes consistant à:
a) introduire un vecteur d'expression dans une cellule hôte eucaryote qui exprime de façon endogène un gène essentiel à la survie cellulaire, ledit vecteur d'expression comprenant au moins
i) un polynucléotide recombinant codant pour un produit qui correspond à et/ou est un variant fonctionnel ou un équivalent fonctionnel du produit d'un gène essentiel à la survie cellulaire qui est exprimé de façon endogène par ladite cellule hôte eucaryote;
ii) un polynucléotide recombinant codant pour un produit qui correspond à et/ou est un variant ou un équivalent du produit d'un gène cible intéressant exprimé par ladite cellule hôte eucaryote;
b) introduire dans ladite cellule hôte un composé induisant un ARNi ciblant l'expression dudit gène cible intéressant exprimé par ladite cellule hôte eucaryote, où ledit composé induisant l'ARNi est sélectionné parmi des petits acides nucléiques interférents (siNA), un petit ARN interférent (siRNA), un microARN (miRNA) et des petits ARN en épingle à cheveux (shRNA), ainsi que leurs précurseurs qui sont traités dans la cellule en composé réel induisant l'ARNi;
c) cultiver les cellules hôtes eucaryotes dans des conditions sélectives, où les conditions sélectives comprennent au moins l'introduction d'au moins un composé induisant l'ARNi, où ledit composé induisant l'ARNi cible au moins le gène essentiel à la survie cellulaire qui est exprimé de façon endogène par ladite cellule hôte eucaryote, et où ledit composé induisant l'ARNi est sélectionné parmi des petits acides nucléiques interférents (siNA), un petit ARN interférent (siRNA), un microARN (miRNA) et des petits ARN en épingle à cheveux (shRNA), ainsi que leurs précurseurs qui sont traités dans la cellule en composé réel induisant l'ARNi.

10. Procédé selon la revendication 9, dans lequel au moins deux des étapes a, b et c ou toutes les étapes sont réalisées en parallèle ou séquentiellement.

11. Procédé selon la revendication 9 ou 10, dans lequel on réalise le procédé de sélection selon au moins l'une des revendications 2 à 6.

12. Kit comprenant au moins:
a) un vecteur d'expression à introduire dans une cellule hôte eucaryote qui comprend
(i) une cassette d'expression comprenant un polynucléotide recombinant codant pour un produit qui correspond à et/ou qui est un variant fonctionnel ou un équivalent fonctionnel d'un gène essentiel à la survie cellulaire qui est exprimé de façon endogène par ladite cellule eucaryote;
et
une autre cassette d'expression comprenant un site de clonage multiple pour insérer un polynucléotide recombinant intéressant;
b) un composé induisant l'ARNi ciblant ledit gène essentiel à la survie cellulaire qui est exprimé de façon endogène par ladite cellule hôte eucaryote, où ledit composé induisant l'ARNi est sélectionné parmi des petits acides nucléiques interférents (siNA), un petit ARN interférent (siRNA), un microARN (miRNA) et des petits ARN en épingle à cheveux (shRNA), ainsi que leurs précurseurs qui sont traités dans la cellule en composé réel induisant l'ARNi.

13. Kit selon la revendication 12, dans lequel ledit vecteur d'expression présente au moins une des caractéristiques suivantes:
a) ledit polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes est un gène de marqueur sélectionnable d'ARNi, où le gène de marqueur sélectionnable d'ARNi est un gène qui confère une résistance à un composé induisant l'ARNi introduit dans la cellule hôte eucaryote et qui permet la sélection de cellules hôtes eucaryotes qui ont incorporé le vecteur d'expression dans les conditions de sélection; et/ou
b) il comprend un autre polynucléotide recombinant codant pour un produit qui correspond à et/ou est un variant fonctionnel ou un équivalent fonctionnel du produit dudit second gène essentiel à la survie cellulaire qui est exprimé de façon endogène par ladite cellule hôte eucaryote; et/ou
c) au moins un polynucléotide recombinant est incorporé dans la cassette d'expression; et/ou
d) au moins un polynucléotide recombinant est incorporé dans la cassette d'expression, où ladite cassette d'expression comprend au moins un des éléments suivants:
i. un promoteur et/ou une séquence activatrice fonctionnelle dans lesdites cellules hôtes eucaryotes ;
ii. un signal de terminaison transcriptionnelle ;
iii. un site poly A ;
iv. un intron ;
v. une séquence leader sécrétoire ;
et/ou
e) il comprend au moins une origine de réplication ; et/ou
f) il comprend au moins un gène marqueur sélectionnable supplémentaire ; et/ou
g) ledit polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes eucaryotes est résistant au composé induisant l'ARNi utilisé pour la sélection.

14. Kit selon la revendication 12 ou 13, dans lequel ledit composé induisant l'ARNi présente une ou plusieurs des caractéristiques suivantes:
a) ledit composé induisant l'ARNi ne cible pas le transcript dudit polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes eucaryotes; et/ou
b) ledit composé induisant l'ARNi cible une région non traduite dudit gène essentiel à la survie cellulaire qui est exprimé de façon endogène par lesdites cellules hôtes eucaryotes; et/ou
c) ledit composé induisant l'ARNi cible une région non traduite dudit gène essentiel à la survie cellulaire qui est exprimé de façon endogène par lesdites cellules hôtes eucaryotes qui n'est pas présente dans le polynucléotide recombinant codant pour un produit qui correspond à et/ou est un variant fonctionnel ou un équivalent fonctionnel du produit codé par ledit gène essentiel à la survie cellulaire qui est exprimé de façon endogène par lesdites cellules hôtes eucaryotes; et/ou
d) ledit gène essentiel à la survie cellulaire exprimé de façon endogène par ladite cellule hôte eucaryote présente au moins l'une des caractéristiques suivantes:
i. son knock-down induit ou favorise l'apoptose; et/ou
ii. son knock-down perturbe le cycle cellulaire;
iii. ledit gène est sélectionné dans le groupe composé de:
| |
|---|
| Ubiquitine |
| Polokinase 1 |
| Topoisomérase 1 |
| Intégrine bêta 1 |
| Homoloque de RAD51 (homol. RecA, E. coli) |
| Exportine 5 |
| Exportine 7 |
| Protéine de liaison RAN 17 |
| Importine 11 |
| Importine 13 |
| Facteur 1 alpha 1 d'élongation de traduction eucaryote |
| Facteur 1 alpha 2 d'élongation de traduction eucaryote |
| Facteur 1 epsilon 1 d'élongation de traduction eucaryote |
et/ou
e) ledit composé induisant l'ARNi est sélectionné dans le groupe composé de :
i. c'est un siRNA, un miRNA ou un shRNA ; et/ou
ii. c'est une molécule double brin de siRNA comportant de préférence 18 à 30 nt ; et/ou
iii. il est exprimé par un vecteur.

15. Utilisation d'un kit selon une ou plusieurs des revendications 12 à 14 pour exprimer un produit intéressant ou pour réaliser des expériences sur l'ARNi.
